(19) Europäisches Patentamt
European Patent Office
Office européen des brevets

(11) Veröffentlichungsnummer: **0 223 184**
**A2**

(12) **EUROPÄISCHE PATENTANMELDUNG**

(21) Anmeldenummer: **86115596.8**

(22) Anmeldetag: **11.11.86**

(51) Int. Cl.⁴: **C 07 D 501/20**, A 61 K 31/545

(30) Priorität: **21.11.85 DE 3541095**

(43) Veröffentlichungstag der Anmeldung: **27.05.87 Patentblatt 87/22**

(84) Benannte Vertragsstaaten: **AT BE CH DE ES FR GB IT LI NL SE**

(71) Anmelder: **BAYER AG, Konzernverwaltung RP Patentabteilung, D-5090 Leverkusen 1 Bayerwerk (DE)**

(72) Erfinder: **Boberg, Michael, Dr., Untere Bergerheide 47, D-5600 Wuppertal 1 (DE)**
Erfinder: **Angerbauer, Rolf, Dr., Sterntalerweg 29, D-5600 Wuppertal 1 (DE)**
Erfinder: **Metzger, Karl-Georg, Dr., Pahlkestrasse 75, D-5600 Wuppertal 1 (DE)**
Erfinder: **Zeller, Hans-Joachim, Dr., Elsbeekerstrasse 46, D-5620 Velbert 15 (DE)**

(54) **Neue Cephalosporine, Verfahren zu deren Herstellung sowie ihre Verwendung als Arzneimittel.**

(57) Die Erfindung betrifft neue Cephalosporine der allgemeinen Formel (I)

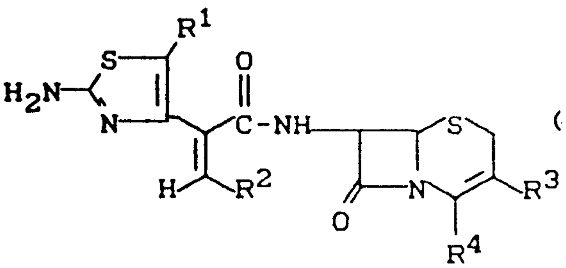

(I),

in welcher die Substituenten $R^1$ bis $R^4$ die in der Beschreibung angegebene Bedeutung haben, Verfahren zu ihrer Herstellung sowie ihre Verwendung in Arzneimitteln, insbesondere in der antibakteriellen Therapie.

EP 0 223 184 A2

0223184

BAYER AKTIENGESELLSCHAFT 5090 Leverkusen, Bayerwerk
Konzernverwaltung RP
Patentabteilung Ad/by-c

# Neue Cephalosporine, Verfahren zu deren Herstellung sowie ihre Verwendung als Arzneimittel

Die Erfindung betrifft neue Cephalosporine, Verfahren zu ihrer Herstellung sowie ihre Verwendung in Arzneimitteln, insbesondere in der antibakteriellen Therapie.

Aus EP-A 49 448 sind Cephalosporine bekannt, die als Acylseitenkette einen unsubstituierten Aminothiazolylrest tragen.

Die vorliegende Erfindung betrifft neue Cephalosporine der allgemeinen Formel (I)

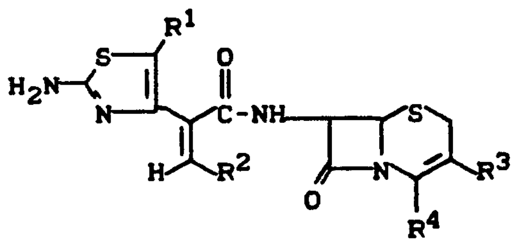

(I),

in welcher

$R^1$ - für Alkyl, Alkenyl, Alkoxy, Alkylthio, Halogen, Trifluormethyl oder Trifluormethoxy steht,

$R^2$ - für Wasserstoff, für Alkenyl, für gegebenenfalls substituiertes Alkyl, gegebenenfalls substituiertes Aryl, gegebenenfalls substituiertes Heterocyclyl, Alkoxy, Carboxy, Alkoxycarbonyl, Halogen oder für eine Gruppe der Formel $-S(O)_n-B-A$ steht,

worin

n - 0, 1 oder 2 bedeutet,

B - eine direkte Bindung, Sauerstoff oder eine Gruppe -N-X darstellt, und

A und X unabhängig voneinander für Wasserstoff, gegebenenfalls substituiertes Alkyl, gegebenenfalls substituiertes Aryl oder gegebenenfalls substituiertes Heterocyclyl stehen, oder gemeinsam einen gegebenenfalls substituierten carbocyclischen oder heterocyclischen Ring bilden,

$R^3$ - für Wasserstoff, Alkyl, Alkenyl, Alkoxy, Alkylthio, Halogen oder für eine Gruppe der Formel $-CH_2-Y$ steht,

wobei

Y - für Hydroxy, Formyloxy, Alkanoyloxy, Aminocarbonyloxy, für gegebenenfalls substituiertes Heterocyclylthio, oder für einen stickstoffhaltigen und über N gebundenen positiv geladenen, bis zu insgesamt 3 Stickstoffatome enthaltenden 6-Ring steht, an den bis zu zwei weitere Ringe ankondensiert sein können und der gegebenenfalls substituiert sein kann,
oder für eine Gruppe der Formel

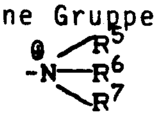

steht, worin

$R^5$, $R^6$ und $R^7$ gleich oder verschieden sein können und einen substituierten oder unsubstituierten Alkylrest oder einen mono- oder bicyclischen, gegebenenfalls substituierten, carbo- oder heterocyclischen Ring bedeuten
oder

$R^5$ einen gegebenenfalls substituierten Alkylrest oder einen mono- oder bicyclischen, substituierten oder unsubstituierten, carbo- oder heterocyclischen Ring darstellt und

$R^6$ und $R^7$ gemeinsam mit dem N-Atom einen gegebenenfalls substituierten mono- oder polycyclischen Ring bilden, der gesättigt oder ungesättigt sein kann und Sauerstoff, Schwefel und/oder Stickstoff als weitere Heteroatome enthalten kann,

und

$R^4$ - je nach der Bedeutung von $R^3$ für $COO^-$ oder COOH steht, und deren Salze.

Die Verbindungen der Formel (I) können als freie Säuren, Ester, als innere Salze oder als nicht-toxische pharmazeutisch verträgliche Salze der sauren Carboxylgruppen, wie Natrium-, Kalium-, Magnesium-, Calcium-, Aluminium- und Ammoniumsalze und nicht-toxische substituierte Ammoniumsalze, mit Aminen wie Di-, Triniedrigalkylaminen, Procain, Dibenzylamin, N,N'-Dibenzylalkylendiamin, N-Benzyl-ß-phenylethylamin, N-Methyl- und N-Ethylmorpholin, 1-Ephenamin, Dehydroabietylamin, N,N'-Bis-dehydroabietylethylendiamin, N-Niedrigalkylpiperidine und andere Aminen, die zur Bildung von Salzen von Penicillinen und Cephalosporinen verwendet werden können, vorliegen.

Die Bedeutung Alkyl umfaßt jeweils geradkettige oder verzweigte, gegebenenfalls substituierte Reste mit bis zu 18 C-Atomen, bevorzugt mit bis zu 10 C-Atomen und speziell mit bis zu 6 C-Atomen, die auch carbocyclisch sein können.

Bevorzugt sind Verbindungen, in denen $R^1$, $R^2$ und $R^4$ die angegebene Bedeutung haben und

**$R^3$ - für Wasserstoff, Alkyl, Alkenyl, Alkoxy, Alkylthio, Halogen oder für eine Gruppe der Formel $-CH_2-Y$ steht, wobei**

**Y - für Hydroxy, Formyloxy, Alkanoyloxy, Aminocarbonyloxy, für gegebenenfalls substituiertes Heterocyclylthio, oder**

**- für einen Pyridiniumrest**

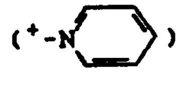

**steht, der gegebenenfalls ein- bis mehrfach gleich oder verschieden substituiert sein kann und an den ein oder zwei gegebenenfalls substituierte 3- bis 7-gliedrige Ringe ankondensiert**

sein können, die jeweils bis zu zwei Heteroatome und bis zu zwei Doppelbindungen enthalten können und auch aromatisch oder heteroaromatisch sein können, oder

- für einen Rest der Formel

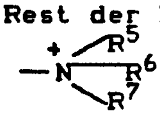

steht, wobei

$R^5$,$R^6$ und $R^7$ gleich oder verschieden voneinander sind und einen gegebenenfalls substituierten $C_1$-$C_6$-Alkylrest oder einen 3- bis 7-gliedrigen gegebenenfalls substituierten Ring darstellen, oder

$R^5$ die obige Bedeutung hat und

$R^6$ und $R^7$ gemeinsam mit dem N-Atom einen 3- bis 7-gliedrigen Ring bilden, der gesättigt oder ungesättigt sein kann und ein- oder mehrfach bevorzugt 1- bis 3-fach gleich oder verschieden substituiert sein kann und ein oder zwei weitere Heteroatome enthalten kann, die Sauerstoff, Stickstoff und/oder Schwefel sein können.

Wenn $R^1$ für Alkyl, Alkoxy oder Alkylthio steht, dann bevorzugt für geradkettiges oder verzweigtes Niederalkyl, Niederalkyloxy oder Niederalkylthio mit jeweils bis zu etwa 6 C-Atomen.

Wenn $R^2$ einen Alkylrest darstellt, dann bevorzugt einen geradkettigen oder verzweigten, gegebenenfalls substituierten Rest mit bis zu 18 C-Atomen, besonders bevorzugt mit bis zu 12 C-Atomen und speziell mit bis zu 6 C-Atomen. Alkyl im Rahmen dieser Definition umfaßt auch carbocyclische Reste.

Wenn der Alkylrest $R^2$ substituiert ist, dann vorzugsweise mit einem oder zwei Substituenten, vorzugsweise aus der Gruppe Halogen, Hydroxy, Niederalkoxy, Oxo, Thio, Nitro, Cyano, Carboxy, Niederalkoxycarbonyl, Aminocarbonyloxy, Sulfo, Aryl, Niederalkanoyloxy, $S(O)_m$-$R^8$ oder

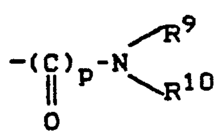

worin

m für 0, 1 oder 2 steht,
p für 0 oder 1 steht,
$R^8$ für Niederalkyl oder Phenyl steht und
$R^9$,$R^{10}$ - unabhängig voneinander Wasserstoff oder gemeinsam oder einzeln für Niederalkyl oder Niederalkanoyl stehen.

Wenn $R^2$ einen Arylrest darstellt, dann bevorzugt einen Rest der Formel

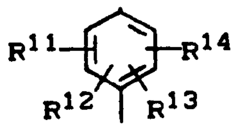

,

worin

$R^{11}$, $R^{12}$, $R^{13}$ und $R^{14}$ unabhängig voneinander Wasserstoff, Halogen, gegebenenfalls substituiertes Niederalkyl, Phenyl, Niederalkanoyl, Amino, Niederalkylamino, Diniederalkylamino, Niederalkanoylamino, Nitro, Cyano, Niederalkoxy, Niederalkylthio, Carboxy, Niederalkoxycarbonyl, Aminocarbonyloxy, Sulfo oder Phenylsulfonyl oder Niederalkylsulfonyl bedeuten.

Wenn $R^2$ einen heterocyclischen Rest darstellt, dann bevorzugt einen 5- oder 6-Ring, mit 1-4 Heteroatomen, gleich oder verschieden aus der Gruppe N, O und S. Besonders bevorzugt sind hier Pyridin-, Pyrimidin-, Pyrazin-, Pyridazin-, Furan-, Thiophen-, Isoxazol-, Thiazol-, Thiadiazol-, Triazol- oder Chinolinreste.

Bevorzugte Substituenten an einem heterocyclischen Rest in $R^2$ sind Niederalkyl, Phenyl, Halogen, Niederalkanoyl, Niederalkoxy, Niederalkylthio, Nitro, Cyano, Amino, Niederalkylamino, Carboxy, Niederalkoxycarbonyl, Aminocarbonyloxy, Sulfo, Phenylsulfonyl, Niederalkylsulfonyl.

Wenn $R^2$ für Alkoxy oder Alkoxycarbonyl steht, dann bevorzugt für Niederalkoxy mit bis etwa 8 C-Atomen und Niederalkoxycarbonyl mit bis etwa 8 C-Atomen.

Wenn $R^2$ für eine Gruppe der Formel $-S(O)_n-B-A$ steht, hat n bevorzugt die Bedeutung von 0, 1 oder 2, B hat bevorzugt die Bedeutung von Sauerstoff, einer direkten Bindung oder von der Gruppe -NX- wobei A und X bevorzugt unabhängig voneinander für Wasserstoff, Alkyl, Aryl oder Heterocyclyl mit der bereits angegebenen Bedeutung stehen, oder A und X bilden gemeinsam bevorzugt einen gegebenenfalls substituierten 5- bis 6-gliedrigen carbocyclischen oder heterocyclischen Ring aus der Gruppe: Pyrrolidin, Piperidin, Piperazin, Morpholin oder Thiomorpholin.

Wenn $R^3$ für Alkyl, Alkenyl, Alkoxy oder Alkylthio steht, dann jeweils für Niederalkyl, Niederalkenyl, Niederalkoxy oder Niederalkylthio mit jeweils bis zu 8 C-Atomen, wobei die Alkylreste sowie der Alkenylrest gerade und verzweigt sein können.

Steht $R^3$ für die Gruppe $-CH_2-Y$ und Y bedeutet gegebenenfalls substituiertes Heterocyclylthio, dann steht Y bevorzugt für über ein Schwefelatom gebundenes Thiadiazolyl, Triazolyl, Triazinon oder Tetrazolyl, wobei als Substituenten gegebenenfalls Niederalkyl, Carboxyniederalkyl, Sulfoniederalkyl, Carbamoylniederalkyl, Sulfonamidoniederalkyl, Hydroxyniederalkyl, Dimethylaminoniederalkyl oder Diethylaminoniederalkyl mit jeweils bis zu 8 C-Atomen im Alkylrest in Frage kommen.

Steht Y in $R^3$ für einen stickstoffhaltigen und über N gebundenen positiv geladenen Ring, dann bevorzugt für einen Pyridiniumrest, der gegebenenfalls ein- oder mehrfach, vorzugsweise 1- bis 3-fach, insbesondere 1- bis 2-fach substituiert ist, beispielsweise durch $C_1$-$C_4$-Alkyl, Halogen, Carbamoyl, N-$C_1$-$C_4$-Alkylcarbamoyl, Hydroxy-$C_1$-$C_4$-alkyl, wie insbesondere Hydroxymethyl, Hydroxyethyl, Hydroxypropyl, Hydroxyisopropyl, Hydroxybutyl, Hydroxy-sec.-butyl oder Hydroxy-tert.-butyl, und wobei auch zwei oder drei Hydroxylgruppen am Alkylrest stehen können, Carboxy-$C_1$-$C_4$-alkyl, wie insbesondere Carboxymethyl und Carboxyethyl, $C_1$-$C_4$-Alkoxycarbonyl-$C_1$-$C_4$-alkyl, wie insbesondere Methoxycarbonylmethyl, Ethoxycarbonylmethyl, Methoxycarbonylethyl, Formyl-$C_1$-$C_4$-alkyl, wie insbesondere Formylethyl, $C_1$-$C_4$-Alkylcarbonyl-$C_1$-$C_4$-alkyl, wie insbesondere Methylcarbonylmethyl, Ethylcarbonylmethyl, Methylcarbonylethyl und Ethylcarbonylethyl, deren beide Alkylgruppen auch noch durch Hydroxy substituiert sein können und deren Carbonylgruppen auch in ketalisierter Form vorliegen können, durch Carbamoyl substituiertes $C_1$-$C_4$-Alkyl, wie insbesondere Carbamoylmethyl und Carbamoylethyl, die am Stickstoff auch noch durch Hydroxy substituiert sein können, wie insbesondere N-Hydroxy-carbamoylmethyl, Sulfo-$C_1$-$C_4$-alkyl, wie insbesondere Sulfoethyl oder 1-Hydroxy-1-sulfomethyl, $C_1$-$C_4$-Alkoxy-$C_1$-$C_4$-alkyl, wie insbesondere Methoxymethyl, Ethoxymethyl, Propoxymethyl, Isopropoxymethyl, Methoxyethyl, Ethoxyethyl, Methoxypropyl und Methoxyisopropyl, die auch durch Hydroxy substituiert sein können, wie insbesondere Hydroxymethoxymethyl und Hydroxyethoxymethyl, $C_1$-$C_4$-Alkylthio-$C_1$-$C_4$-alkyl, wie insbesondere Methylthiomethyl,

Ethylthiomethyl, Methylthioethyl und Ethylthioethyl, $C_1$-$C_4$-Alkylsulfinyl-$C_1$-$C_4$-alkyl, wie insbesondere Methylsulfinylmethyl, Ethylsulfinylmethyl, Methylsulfinylethyl und Ethylsulfinylethyl, $C_1$-$C_4$-Alkylsulfonyl-$C_1$-$C_4$-alkyl, wie insbesondere Methylsulfonylmethyl, Ethylsulfonylmethyl, Methylsulfonylethyl und Ethylsulfonylethyl, $C_3$-Alkenyloxy-$C_1$-$C_4$-alkyl, wie insbesondere Allyloxymethyl und Allyloxyethyl, $C_3$-Alkenylthio-$C_1$-$C_4$-alkyl, wie insbesondere Allylthiomethyl, $C_3$-Alkenylsulfinyl-$C_1$-$C_4$-alkyl, wie insbesondere Allylsulfinylmethyl, $C_3$-Alkenylsulfonyl-$C_1$-$C_4$-alkyl, wie insbesondere Allylsulfonylmethyl, Cyano-$C_1$-$C_3$-alkyl, wie insbesondere Cyanomethyl und Cyanoethyl, Epoxy-$C_1$-$C_3$-alkyl, wie insbesondere Epoxyethyl und Epoxypropyl, Trifluormethyl, Hydroxyiminomethyl und $C_1$-$C_3$-Alkyloximinomethyl, wie insbesondere Methoxyiminomethyl, $C_3$-$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl, $C_3$-Alkinyl, wie insbesondere Propargyl, $C_3$-$C_6$-Cycloalkyl und $C_3$-$C_6$-Cycloalkyl-methyl, wie insbesondere Cyclopropyl, Cyclobutyl, Cyclopentyl, Cyclohexyl und Cyclopentylmethyl, wobei die Ringe auch substituiert sein können, z.B. durch Hydroxy, wie insbesondere 1-Hydroxy-1-cyclopentyl und 1-Hydroxy-1-cyclohexyl, oder durch Halogen, vorzugsweise Chlor, durch Carboxy, $C_1$-$C_4$-Alkoxycarbonyl oder Cyano, $C_5$-$C_6$-Cycloalkenyl, wie insbesondere Cyclopenten-1-yl und Cyclohexen-1-yl, $C_1$-$C_4$-Alkoxy, wie insbesondere Methoxy, Ethoxy, Propoxy, Isopropoxy, Butoxy, Isobutoxy und tert.-Butoxy, vorzugsweise Methoxy, wobei diese Alkoxygruppen auch noch substituiert sein können, beispielsweise durch Hydroxy, Carboxy oder $C_1$-$C_4$-Alkyloxycarbonyl, insbesondere

Carboxymethyloxy und Methyloxycarbonylmethyloxy, Epoxy-$C_2$-$C_3$-alkyloxy, wie insbesondere Epoxyethoxy oder Epoxypropoxy, $C_3$-Alkenyloxy, wie insbesondere Allyloxy, $C_3$-Alkinyloxy, wie insbesondere Propargyloxy, Aryloxy, wie insbesondere Phenoxy, Amino, $C_1$-$C_5$-Alkylamino, wie insbesondere Ethylamino, $C_1$-$C_5$-Dialkylamino, wie insbesondere Dimethylamino und Diethylamino, $C_1$-$C_4$-Alkoxycarbonylamino, wie insbesondere Methoxycarbonylamino und Ethoxycarbonylamino, $C_1$-$C_4$-Alkylcarbonylamino, wie insbesondere Methylcarbonylamino, N-$C_1$-$C_4$-Alkyl- und Dialkylcarbamoylamino, wie insbesondere N-Methylcarbamoylamino, N,N-Diethylcarbamoylamino, $C_1$-$C_4$-Alkylsulfonylamino, wie insbesondere Methyl- oder Ethylsulfonylamino, Cyano, Hydroxy, insbesondere 3-Hydroxy, $C_1$-$C_4$-Alkylthio, wie insbesondere Methylthio, Ethylthio, Propylthio und Isopropylthio, die auch substituiert sein können durch Hydroxy, insbesondere Hydroxyethylthio, $C_1$-$C_4$-Alkylsulfinyl, wie insbesondere Methylsulfinyl, Ethylsulfinyl, Propylsulfinyl und Isopropylsulfinyl, die auch substituiert sein können durch Hydroxy, insbesondere Hydroxyethylsulfinyl, $C_1$-$C_4$-Alkylsulfonyl, wie Methyl- oder Ethyl- oder Propyl- oder Isopropylsulfonyl, die auch substituiert sein können durch Hydroxy, insbesondere Hydroxyethylsulfonyl, Carboxymethylthio und $C_1$-$C_4$-Alkoxycarbonylmethylthio, insbesondere Methoxycarbonylmethylthio, Carboxymethyl-sulfinyl und -sulfonyl, sowie $C_1$-$C_4$-Alkoxycarbonylmethylsulfinyl und -sulfonyl, insbesondere Methoxycarbonylmethyl-sulfinyl und -sulfonyl, $C_3$-Alkenylthio, wie Allylthio und Propen-1-yl-thio, $C_3$-Alkenylsulfinyl, wie Allylsulfinyl und Propen-1-yl-sulfinyl, $C_3$-Alkenylsulfonyl, wie Allylsulfonyl und Propen-1-yl-sulfonyl, Phenyl und Benzyl,

die auch substituiert sein können, beispielsweise durch Halogen, insbesondere Chlor, wie z.B. 4-Chlorbenzyl, 2'-Thienyl und 3'-Thienyl, Formyl und ketalisiertes Formyl, wie z.B. 1,3-Dioxolan-2-yl, $C_1$-$C_4$-Alkylcarbonyl, insbesondere Acetyl und Propionyl, vorzugsweise Acetyl, die auch durch Hydroxy substituiert sein und in ketalisierter Form vorliegen können, wie z.B. 2-Methyl-1,3-dioxolan-2-yl, Benzoyl, $C_1$-$C_4$-Alkylcarbonylamino, insbesondere Acetylamino und Propionylamino, Formylamino, Carboxy, $C_1$-$C_4$-Alkoxycarbonyl, insbesondere Methoxycarbonyl und Ethoxycarbonyl,

und an den ein gegebenenfalls substituierter 5- oder 6-gliedriger Ring ankondensiert sein kann, der bis zu zwei Heteroatome, bevorzugt aus der Gruppe O, N und S, und bis zu zwei Doppelbindungen enthalten kann und der auch aromatisch oder heteroaromatisch sein kann. Als ankondensierte Ringe kommen insbesondere folgende Ringsysteme in Betracht:

Cyclopenteno, Dihydrocyclopenteno, Cyclohexeno, Dihydrocyclohexeno, Benzo, Furo, Dihyrofuro, Pyrano, Dihydropyrano, Thieno, Dihydrothieno, Thiopyrano, Dihydrothiopyrano, Pyrido, Dihydropyrido, Tetrahydropyrido, Pyrimido, Dihydropyrimido, Tetrahydropyrimido, Pyrazino, Dihydropyrazino, Tetrahydropyrazino, Pyridazino, Dihydropyridazino, Tetrahydropyridazino,

die jeweils ein- oder mehrfach, vorzugsweise jedoch einfach substituiert sein können, vorzugsweise durch

$C_1$-$C_4$-Alkyl, wie insbesondere Methyl, Ethyl und Isopropyl, $C_3$-$C_6$-Cycloalkyl, wie insbesondere Cyclopropyl, $C_1$-$C_4$-Alkoxy, wie insbesondere Methoxy, Ethoxy, $C_1$-$C_3$-Hydroxyalkyl, wie insbesondere Hydroxymethyl, Hydroxyethyl, Halogen, wie insbesondere Chlor und Fluor, Hydroxy, Carboxy und Cyano, $C_1$-$C_6$-Alkoxycarbonyl, wie insbesondere Methoxycarbonyl und Ethoxycarbonyl, Oxo und Hydroximino, Carbamoyl und Sulfamoyl, Amino, $C_1$-$C_4$-Alkylamino, wie insbesondere Methylamino und Ethylamino, $C_1$-$C_4$-Dialkylamino, wie insbesondere Diethylamino.

Wenn Y für die Gruppe

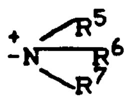

steht und $R^5$ und/oder $R^6$ und/oder $R^7$ einen substituierten Alkylrest darstellen, dann bevorzugt mit einem oder zwei Substituenten aus der Reihe Hydroxy, Carboxy, Niederalkoxycarbonyl, Aminocarbonyl, Niederalkylaminocarbonyl, Formyl oder Niederalkanoyl, deren Carbonylgruppe auch in ketalisierter Form vorliegen können, Oxo, Thio, Sulfo, Cyano, Nitro, Amino, Halogen, Niederalkylamino, Diniederalkylamino, Niederalkanoylamino, Aminocarbonylamino, Niederalkylsulfonyl, Phenyl, Heterocyclyl oder Heteroaryl.

Wenn $R^5$ und/oder $R^6$ und/oder $R^7$ einen gesättigten oder ungesättigten gegebenenfalls substituierten 3- bis 7-gliedrigen Ring darstellen, dann bevorzugt einen carbo- oder heterocyclischen Ring, der bis zu drei, bevorzugt ein oder zwei Heteroatome enthalten kann, die Sauerstoff, Stickstoff und/oder Schwefel sein können.

Ist der Ring substituiert, dann bevorzugt durch einen oder zwei Substituenten aus der Reihe Niederalkyl, Hydroxy, Hydroxyniederalkyl, Carboxy, Niederalkoxycarbonyl, Formyl oder Niederalkanoyl, deren Carbonylgruppen auch in ketalisierter Form vorliegen können, Carbamoyl, Sulfo, Cyano, Nitro, Amino, Halogen, Niederalkylamino, Diniederalkylamino, Niederalkanoylamino, Niederalkoxy, Niederalkylthio, Niederalkylsulfinyl, Niederalkylsulfonyl, Phenyl, Heterocyclyl oder Heteroaryl.

Wenn $R^6$ und $R^7$ gemeinsam mit dem N-Atom einen gegebenenfalls substituierten heterocyclischen Ring bilden, dann bevorzugt einen 3- bis 7-gliedrigen Ring, der ein oder zwei Doppelbindungen und bis zu zwei weitere Heteroatome enthalten kann, die Sauerstoff, Stickstoff oder Schwefel sein können und an den ein weiterer 5- bis 6-gliedriger Ring ankondensiert sein kann.

Wenn der durch $R^6$ und $R^7$ gemeinsam mit dem N-Atom gebildete heterocyclische Ring substituiert ist, dann vorzugsweise mit einem oder zwei Substituenten aus der Reihe Niederalkyl, Hydroxy, Hydroxyniederalkyl, Carboxy, Niederalkoxycarbonyl, Formyl oder Niederalkanoyl, deren Carbonylgruppen auch in ketalisierter Form vorliegen können, Sulfamoyl, Carbamoyl, Sulfo, Cyano, Nitro, Amino, Halogen, Niederalkylamino, Diniederalkylamino, Niederalkanoylamino, Niederalkoxy, Niederalkylthio, Niederalkylsulfinyl, Niederalkylsulfonyl, Aryl, Heteroaryl oder Heterocyclyl.

Die Bedeutung von Aryl im Rahmen dieser Definition ist die bereits für $R^2$ angegebene.

Die Reste Heterocyclyl und Heteroaryl im Rahmen dieser Definition bedeuten bevorzugt: Pyridyl, Pyrimidyl, Pyridazinyl, Pyrazinyl, Thienyl, Furyl, Isoxazolyl, Thiazolyl, Piperazinyl, Piperidinyl, Pyrrolidinyl, Morpholinyl, Imidazolyl oder Chinolyl.

Halogen im Rahmen der Erfindung bedeutet bevorzugt Fluor, Chlor oder Brom.

Besonders bevorzugt sind Verbindungen der Formel (I), in welcher

$R^1$ - für $C_1$-$C_4$-Alkyl, $C_1$-$C_4$-Alkoxy, Fluor, Chlor, Brom oder Trifluormethyl steht,

$R^2$ - für $C_1$-$C_4$-Alkyl steht, das gegebenenfalls substituiert ist durch Hydroxy, Fluor, Chlor, Carboxy, $C_1$-$C_4$-Alkoxycarbonyl, $C_1$-$C_4$-Alkylsulfonyl oder Carbamoyloxy oder
- für Cyclopentyl oder Cyclohexyl steht, oder
- für Phenyl steht, das gegebenenfalls substituiert ist durch Fluor, Chlor, $C_1$-$C_4$-Alkyl, $C_1$-$C_4$-Alkoxy, $C_1$-$C_4$-Alkylthio, Carboxy, $C_1$-$C_4$-Alkoxycarbonyl, Di-$C_1$-$C_4$-Alkylamino, Cyano, Nitro oder Sulfo oder
- für gegebenenfalls durch $C_1$-$C_4$-Alkyl, Fluor, Chlor, Amino, Di-$C_1$-$C_4$-Alkylamino oder Acetylamino substituiertes Pyridyl, Thienyl, Pyrimidyl, Thiazolyl, Diazolyl oder Thiadiazolyl steht, oder
- für $C_1$-$C_4$-Alkoxy, Carboxy, $C_1$-$C_4$-Alkoxycarbonyl, Fluor, Chlor, $C_1$-$C_4$-Alkylsulfonyl oder Phenylsulfonyl steht,

$R^3$ - für Wasserstoff, $C_1$-$C_4$-Alkyl, $C_1$-$C_4$-Alkoxy, $C_2$-$C_4$-Alkenyl, $C_1$-$C_4$-Alkylthio, Fluor, Chlor, Brom oder für eine Gruppe der Formel $-CH_2-Y$ steht,

wobei

Y - für Hydroxy, Formyloxy, Acetyloxy, Methoxy, Aminocarbonyloxy, für gegebenenfalls durch Methyl, Carboxymethyl, Sulfomethyl, Dimethylaminoethyl oder Sulfamoylethyl substituiertes Triazolyl, Thiadiazolyl, Triazinon oder Tetrazolyl steht, oder

\- für einen Pyridiniumrest steht, an den gegebenenfalls ein 5- bis 6-gliedriger Ring aus der Reihe Cyclopenteno, Cyclohexeno oder Benzo ankondensiert ist und der gegebenenfalls ein- oder mehrfach substituiert ist durch Fluor, Chlor, Methyl, Ethyl, Carbamoyl, Hydroxymethyl, Hydroxyethyl, Hydroxypropyl, Carboxymethyl, Carboxyethyl, Methoxycarbonylmethyl, Ethoxycarbonylmethyl, Methoxycarbonylethyl, Formylmethyl, Formylethyl, Methylcarbonylmethyl, Carbamoylmethyl, Carbamoylethyl, Sulfomethyl, Sulfoethyl, Methoxymethyl, Ethoxymethyl, Methoxyethyl, Methylthiomethyl, Methylsulfinylmethyl, Methylsulfinylethyl, Methylsulfonylmethyl, Methylsulfonylethyl, Ethylsulfonylmethyl, Allyloxymethyl, Cyanomethyl, Cyanoethyl, Trifluormethyl, Allyl, Cyclopropyl, Cyclopentyl, Cyclohexyl, Cyclopentylmethyl, Methoxy, Ethoxy, Propoxy, Isopropoxy, Carboxymethoxy, Allyloxy, Phenoxy, Amino, Methylamino, Ethylamino, Dimethylamino, Diethylamino, Methoxycarbonylamino, Acetylamino,

Methylsulfonylamino, Cyano, Hydroxy, Methylthio, Methylsulfinyl, Methylsulfonyl, Ethylsulfonyl, Propylsulfonyl, Phenyl, Benzyl, Chlorbenzyl, Chlorphenyl, Formyl, Acetyl, Formylamino, Carboxy, Methoxycarbonyl oder Ethoxycarbonyl, oder

- für eine Gruppe der Formel

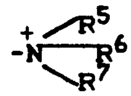 steht,

wobei

$R^5$,$R^6$ und $R^7$ gleich oder verschieden sind und einen $C_1$-$C_6$-Alkylrest, wie insbesondere Methyl, Ethyl, Propyl, Cyclopropyl, Cyclopropylmethyl, Cyclopentyl, Cyclopentylmethyl, oder einen substituierten $C_1$-$C_6$-Alkylrest, wie insbesondere Hydroxymethyl, Hydroxyethyl, Hydroxypropyl, Aminomethyl, Aminoethyl, Hydroxycarbonylmethyl, Hydroxycarbonylethyl, Cyanomethyl, Nitromethyl, Nitroethyl, Methoxymethyl, Methoxycarbonylmethyl, Trifluormethyl darstellen,

oder in der

$R^5$ - die obige Bedeutung hat und

$R^6$ und $R^7$ gemeinsam mit dem N-Atom einen 5- bis 6-gliedrigen heterocyclischen Ring bilden, der ein weiteres Heteroatom enthalten kann und an den ein weiterer Ring ankondensiert

sein kann wie insbesondere Pyrrolidinium, Piperazinium, Piperidinium, Morpholinium, Pyrrolinium, Pyrazolidinium, Indolinium, Isoindolinium, Oxazolidinium, Thiazolidinium, Thiomorpholinium und der gegebenenfalls substituiert sein kann durch $C_1$-$C_4$-Alkyl, wie insbesondere Methyl, Ethyl, Propyl, das wiederum substituiert sein kann, z.B. durch Hydroxy, Carboxy, Cyano, Nitro, Amino, Halogen, Alkoxy oder $C_1$-$C_6$-Alkylcarbonyl, wie insbesondere Formyl, Methyl- oder Ethylcarbonyl, durch Carbamoyl, Sulfo, Cyano, Nitro, Halogen, wie insbesondere Fluor und Chlor, Amino, $C_1$-$C_6$-Alkyl- und Dialkylamino, wie insbesondere Methylamino, Dimethylamino, Diethylamino, $C_1$-$C_6$-Alkylcarbonylamino, wie insbesondere Methylcarbonylamino und Ethylcarbonylamino, $C_1$-$C_6$-Alkoxy, wie insbesondere Methoxy, $C_1$-$C_6$-Alkylthio, wie insbesondere Methylthio, $C_1$-$C_6$-Alkylsulfinyl, wie insbesondere Methylsulfinyl, $C_1$-$C_6$-Alkylsulfonyl, wie insbesondere Methyl- und Ethylsulfonyl, durch Aryl wie insbesondere Phenyl oder Naphthyl, die auch substituiert sein können, durch Hetaryl, wie Pyridyl, das auch substituiert sein kann

und

$R^4$ - je nach der Bedeutung von $R^3$ für COOH oder $COO^-$ steht,

und deren Salze.

Ganz besonders bevorzugt sind Verbindungen der Formel (I), in welcher

$R^1$ - für Methyl, Fluor, Chlor oder Trifluormethyl steht,

$R^2$ - für Methyl, Ethyl, Propyl, Isopropyl, Trifluormethyl, Carboxymethyl, Methoxycarbonylmethyl, Cyclopentyl, Cyclohexyl steht, oder

- für Phenyl, Chlorphenyl, Carboxyphenyl,
- für Pyridyl oder Aminothiazolyl,
- für Carboxy,
- für Methoxy, Ethoxy, Methylthio, Methylsulfonyl, Ethylsulfonyl, Phenylsulfonyl, Fluor oder Chlor steht,

$R^3$ - für Wasserstoff, Chlor, Fluor, Methyl, Methoxy, Methylthio, Trifluormethyl, Vinyl oder für die Gruppe der Formel $-CH_2-Y$ steht,

worin

Y - für Methoxy, Methylcarbonyloxy, Carbamoyloxy oder einen Rest der Formel

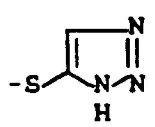 , 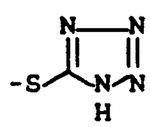 , 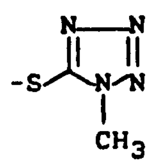 , 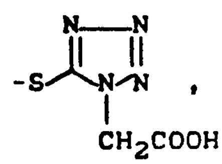 ,

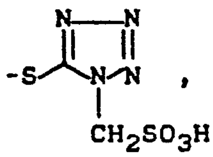 , 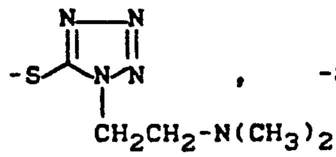 , 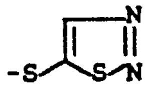 ,

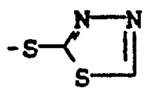 , 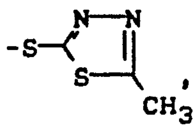 , 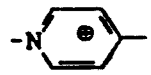 , 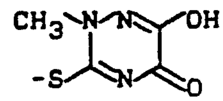 ,

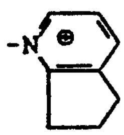 , 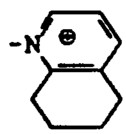 , 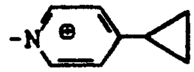 , 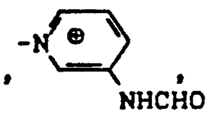 ,

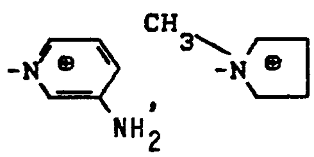 , 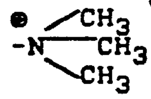 , 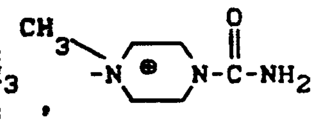 ,

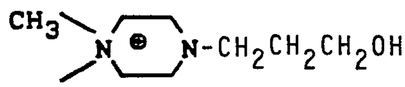

und

$R^4$ - für COOH oder $COO^-$ steht,

und deren Salze.

Die Verbindungen der allgemeinen Formel (I) erhält man, wenn man Verbindungen der allgemeinen Formel (II)

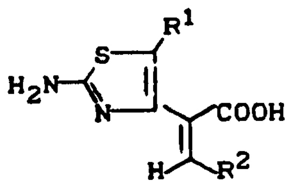

(II),

in welcher

$R^1$ und $R^2$ die angegebene Bedeutung haben,

in denen die Aminogruppe geschützt oder ungeschützt vorliegen kann, nach Aktivierung der Carboxylgruppe durch Überführung in ein gemischtes Anhydrid, beispielsweise mit Chlorameisensäureethylester oder Methansulfonsäurechlorid, nach Überführung in das Säurehalogenid oder nach Überführung in einen aktivierten Ester mit beispielsweise N-Hydroxybenztriazol und Dicyclohexylcarbodiimid, mit Verbindungen der allgemeinen Formel (III),

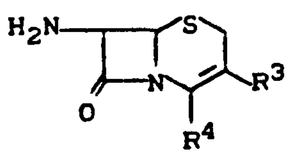

(III),

in welcher

$R^3$ und $R^4$ die oben angegebene Bedeutung haben,

zur Umsetzung gebracht werden, dann gegebenenfalls Schutzgruppen abgespalten werden und die gewünschten Salze oder aus Salzen die freien Säuren hergestellt werden.

Für die Kupplung von Carbonsäuren (II) an β-Lactame der Formel (III) kann eine große Zahl von aus der Cephalosporin- bzw. Penicillinchemie bekannten Methoden verwendet werden. Es hat sich als vorteilhaft erwiesen, die Carbonsäuren der allgemeinen Formel (II) ohne Amin-Schutzgruppe zu aktivieren und dann mit den β-Lactamen der Formel (III), die als Salze mit einem Amin in Lösung gebracht wurden, zu kuppeln. Besonders vorteilhaft ist die Aktivierung mit Sulfonsäurederivaten der Formel (IV) zu Anhydriden der Formel (V)

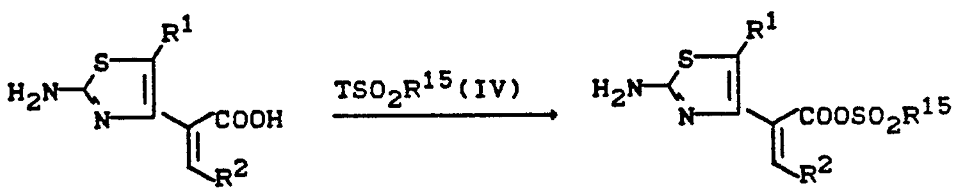


(II) (V)

worin

T - einen Rest $R^{15}$-$SO_2O$ oder Halogen darstellt und

$R^{15}$- einen Alkylrest mit 1 bis 10 C-Atomen darstellt, der gegebenenfalls durch Fluor, Chlor, Cyano, Phenyl, Alkoxycarbonyl, Alkoxy oder Alkyl substituiert sein kann, wobei letztere Alkylreste 1 bis 4 C-Atome tragen

können, oder ein Phenylrest, der gegebenenfalls durch Fluor, Chlor, Brom, Cyano, Alkyl, Alkoxy, Alkylthio, Alkylcarbonyl - wobei letztere Alkylgruppen 1 bis 4 C-Atome tragen können - Nitro, Trifluormethyl und Phenyl substituiert sein kann.

Wenn $R^{15}$ substituiert ist, sind vorzugsweise 1 bis 3 Substituenten, vorzugsweise die genannten vorhanden.

Ganz besonders bevorzugt stellt $R^{15}$ einen Methyl- oder p-Tolylrest dar.

Die gemischten Anhydride der Formel (V) werden hergestellt, indem man die Carbonsäuren der Formel (II) und 1-1,4 Äquivalente eines Amins in einem Lösungsmittel löst und mit 1 bis 1,2 Äquivalenten eines Sulfonsäurederivates der Formel (IV) reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform oder Dimethylformamid.

Als Amin eignen sich tertiäre Amine wie z.B. Triethylamin, Tripropylamin oder Tributylamin, aber auch sterisch gehinderte sekundäre Amine, wie z.B. Diisopropylamin, sowie Gemische dieser Amine.

Die Umsetzungen können bei Temperaturen zwischen -80°C und Raumtemperatur durchgeführt werden, wobei tiefe Temperaturen eine Isomerisierung der Substituenten an der Doppelbindung vermeiden. Vorteilhaft wird die Aktivierung mit $Cl-SO_2-CH_3$, in Dimethylformamid bei -40 bis -60°C innerhalb von 0,2 bis 24 Stunden, vorzugsweise 0,5 bis 5 Stunden, durchgeführt.

Zum Lösen der Verbindungen der Formel (III) können die bei der Herstellung der Verbindungen der Formel (V) genannten Lösungsmittel oder auch Wasser, sowie als Base die dort genannten Amine verwendet werden.

Besonders vorteilhaft ist auch eine Aktivierung der Carbonsäuren der allgemeinen Formel (II) durch Überführung in einen aktivierten Ester mit beispielsweise N-Hydroxysuccinimid und Dicyclohexylcarbodiimid oder 1-Hydroxybenztriazol und Dicyclohexylcarbodiimid.

Als Lösungsmittel eignen sich alle Solventien, die sich auch für die Herstellung von Anhydriden der Formel (V) eignen.

Die Umsetzungen können bei Temperaturen zwischen -30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann von ausgefallenem Dicyclohexylharnstoff abgesaugt und mit einer Verbindung der Formel (III) in Form einer Lösung ihres Aminsalzes innerhalb von 2 bis 24 Stunden umgesetzt. Zum Lösen der Verbindungen der Formel (III) können die bei der Herstellung der Verbindungen der Formel (V) genannten Lösungsmittel oder auch Wasser sowie als Base die dort genannten Amine verwendet werden.

Die Verbindungen der allgemeinen Formel (IIIa)

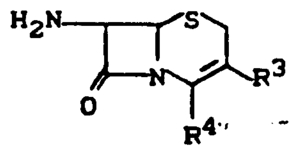

(IIIa)

in welcher

$R^3$ - für Wasserstoff, Alkyl, Alkenyl, Alkoxy, Alkylthio, Halogen oder für die Gruppe der Formel $-CH_2-Y$ steht, wobei

Y - für Hydroxy, Formyloxy, Alkanoyloxy, Aminocarbonyloxy, für gegebenenfalls substituiertes Heterocyclylthio oder
- für einen stickstoffhaltigen und über N-gebundenen positiv geladenen, bis zu insgesamt 3 Stickstoffatome enthaltenen 6-Ring steht, an den bis zu zwei weitere Ringe ankondensiert sein können und der gegebenenfalls substituiert sein kann,

und

$R^4$ - für $COO^-$ oder COOH steht

sind bekannt oder können nach bekannten Methoden hergestellt werden [C.W. Ryan et.al., J. Med. Chem. 12, 310, US-PS 3 925 372, US-PS 3 994 884, DE-OS 25 06 194, EP-OS 144 032, DE-OS 3 013 545 und EP 65 748].

Die Verbindungen der allgemeinen Formel (IIIb), in welcher

$R^3$ - für die Gruppe $-CH_2-Y$ steht und

Y - für einen Rest der Formel

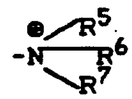 steht,

worin

$R^5$, $R^6$ und $R^7$ gleich oder verschieden sein können und einen substituierten oder unsubstituierten Alkylrest oder einen mono- oder bicyclischen, gegebenenfalls substituierten carbo- oder heterocyclischen Ring bedeuten oder
$R^5$ einen gegebenenfalls substituierten Alkylrest oder einen mono- oder bicyclischen substituierten, carbo- oder heterocyclischen Ring darstellt und
$R^6$ und $R^7$ gemeinsam mit dem N-Atom einen gegebenenfalls substituierten mono- oder polycyclischen Ring bilden, der gesättigt oder ungesättigt sein kann und Sauerstoff, Schwefel oder Stickstoff als weitere Heteroatome enthalten kann,

und

$R^4$ - für $COO^-$ steht,

erhält man, indem man aus Verbindungen der Formel (VI)

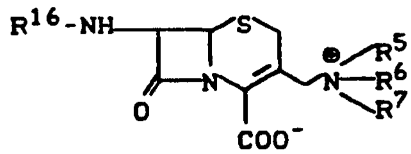

(VI),

in welcher

$R^5$, $R^6$ und $R^7$ die angegebene Bedeutung hat, die Aminoschutzgruppe $R^{16}$ abspaltet (VI → III). Dabei kann $R^{16}$ entweder eine säurelabile Schutzgruppe wie die tert.Butoxycarbonylgruppe oder vorteilhaft eine enzymatisch abspaltbare Schutzgruppe sein. Bevorzugte enzymatisch abspaltbare Schutzgruppen sind Phenacetyl oder 2-Thienylacetyl. Die enzymatische Abspaltung erfolgt bei Raumtemperatur in Wasser oder einem Gemisch aus Wasser und einem polaren organischen Lösungsmittel wie z.B. Acetonitril oder Tetrahydrofuran mit immobilisierter Penicillin-G-Acylase bei pH 7-8, bevorzugt bei pH 7,5-7,8. Während der Abspaltung wird der pH-Wert durch Zugabe einer Base wie Lithiumhydroxid, Natriumhydroxid, Kaliumhydroxid, oder eines tertiären Amins z.B. Triethylamin, Tripropylamin, Tributylamin oder Pyridin konstant gehalten.

Die Verbindungen der Formel (VI) lassen sich aus Estern der Formel (VII) über Zwischenverbindungen der Formel (VIII) herstellen.

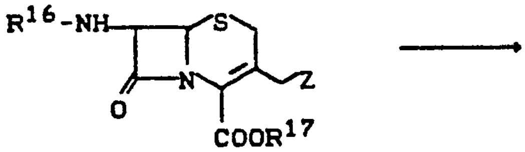

(VII)

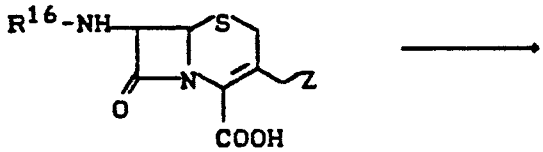

(VI)

(VIII)

In den Estern der Formel (VII) stellt Z eine Fluchtgruppe, wie Mesylat, Tosylat, Brosylat, Triflat, Nonaflat, Iodid, Bromid, Chlorid und $R^{17}$ eine in der Cephalosporinchemie übliche Säureschutzgruppe, bevorzugt eine sauer abspaltbare Schutzgruppe, wie z.B. Benzhydryl, Bis-(4-Methoxyphenyl)-methyl, tert.-Butyl dar.

Die Verbindungen der Formel (VII) werden durch Abspaltung der Säureschutzgruppe $R^{17}$ in die reaktiven freien Säuren der Formel (VIII) überführt. Bei den bevorzugten säurelabilen Schutzgruppen $R^{17}$ wird die Schutzgruppe in einem organischen Lösungsmittel abgespalten. Bevorzugt ist die Abspaltung der Benzhydrylschutzgruppe in Methylenchlorid

mit Trifluoressigsäure, möglicherweise unter Zusatz eines Alkoxybenzols, bevorzugt Methoxybenzol. Die Abspaltung erfolgt bei -20° C bis +30° C, bevorzugt bei 0° C innerhalb von 5 Minuten bis einer Stunde, bevorzugt innerhalb von 20 Minuten.

Die Säure der Formel (VIII) kann nach Abspaltung der Schutzgruppe isoliert werden. Vorteilhaft wird jedoch nicht isoliert, sondern direkt und ohne Reinigung zu Verbindungen der Formel (VI) umgesetzt. Dazu wird die bei der Reaktion (VII → VIII) entstehende Lösung von (VIII) schonend im Vakuum eingeengt. Die zurückbleibende rohe Säure wird in einem organischen Lösungsmittel, bevorzugt in Tetrahydrofuran, aufgenommen und mit 2-20 Äquivalenten, bevorzugt 5-10 Äquivalenten, eines tert. Amins der Formel

$$\begin{array}{l} R^5 \\ | \\ N-R^6 \\ | \\ R^7 \end{array}$$

in der $R^5$, $R^6$ und $R^7$ die vorstehend genannte Bedeutung haben, zu Verbindungen der Formel (VI) umgesetzt. Die Umsetzung wird bei Temperaturen zwischen -20° C und 40° C, bevorzugt bei 25° C, innerhalb von 10 Minuten bis zwei Stunden, bevorzugt innerhalb von 30 Minuten, durchgeführt. Das Produkt kann nach Beendigung der Reaktion durch Zugabe von Diethylether ausgefällt werden. Das so erhalten Rohprodukt kann an einem Harz wie Diaion HP 20 oder XAD 7 gereinigt werden. Es ist auch vorteilhaft möglich, das Rohprodukt direkt zu Verbindungen der Formel (III) weiter umzusetzen.

Die Verbindungen der Formel (VI) lassen sich alternativ auch aus Säuren der Formel (IX) herstellen, in denen $R^{16}$ die vorstehend

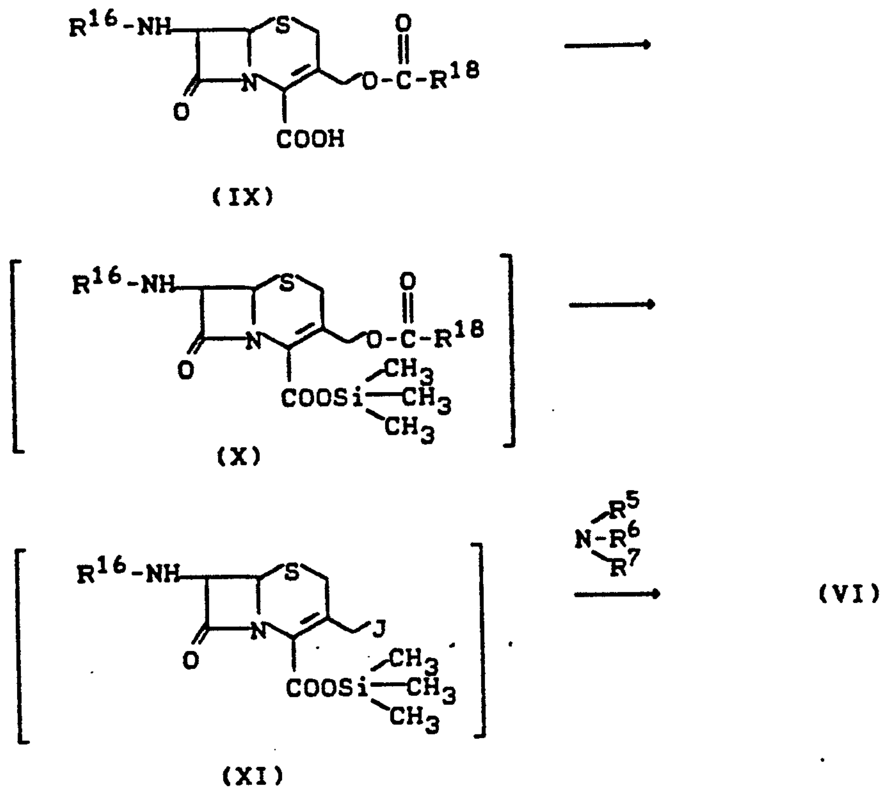

(IX)

(X)

(XI)

(VI)

genannte Bedeutung hat und $R^{18}$ ein gegebenenfalls substituiertes Alkyl oder Aryl darstellt, wie Methyl, Ethyl, Propyl, Chlormethyl, Dichlormethyl, Trichlormethyl, Trifluormethyl, Phenyl. Ganz besonders bevorzugt stellt $R^{18}$ eine Methylgruppe dar.

Die Ausgangsverbindungen der Formel (IX) werden in einem geeigneten organischen Lösungsmittel suspendiert und durch Silylierung zum Silylester X in Lösung gebracht. Besonders geeignete organische Lösungsmittel sind Chloroform, Methylenchlorid, Dichlorethan. Die Silylierung wird mit einem üblichen Silylierungsmittel wie Trimethylchlorsilan (TMCS), Hexamethyldisilazan (HMDS), N,O-Bis(trimethylsilyl)-acetamid (BSA), N,O-Bis(trimethylsilyl)-trifluoracetamid (BSTFA), N-Methyl-N-trimethylsilylacetamid (MSA), N-Methyl-N-trimethylsilyltrifluoracetamid (MSTFA), 1,3-Bis(trimethylsilyl)harnstoff, Trimethylsilyltrifluoromethansulfonat durchgeführt. Dabei können auch mehrere Silylierungsmittel im Gemisch eingesetzt werden.

Die Silylierung erfolgt bei -30°C bis +70°C, bevorzugt bei -10°C bis +10°C, innerhalb von 5 Minuten bis 30 Minuten. Vorteilhaft wird ein bis zu zehnfacher Überschuß des Silylierungsmittels eingesetzt, bevorzugt ein zwei- bis fünffacher Überschuß.

Die so erhaltene Lösung des Trimethylsilylesters der Formel (X) wird bei -40°C bis +30°C, bevorzugt bei -10°C bis +10°C mit ein bis zehn Äquivalenten, bevorzugt mit drei bis vier Äquivalenten eines Trialkylsilyliodids, besonders bevorzugt Trimethylsilyliodid, innerhalb von 15 Minuten bis 2 Stunden, bevorzugt innerhalb von 30 Minuten bis 1 Stunde, zu Verbindungen der Formel (XI) umgesetzt.

Die Verbindungen der Formel (XI) werden vorteilhaft nicht isoliert, sondern direkt ohne Reinigung mit Aminen

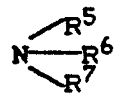

zu Verbindungen der Formel (VI) umgesetzt.

Die als Ausgangsverbindungen verwendeten Stoffe der allgemeinen Formel (II) sind neu und ebenfalls Gegenstand der Erfindung.

Die Verbindungen der Formel (IIa), in welcher $R^1$ für Alkyl oder Trifluormethyl steht und $R^2$ die angegebene Bedeutung hat, erhält man, wenn man Verbindungen der allgemeinen Formel (XII)

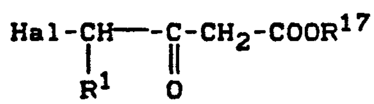

(XII)

in welcher

Hal - für Chlor oder Brom steht,

$R^{17}$ - für verzweigtes oder unverzweigtes, gegebenenfalls substituiertes $C_1$-$C_4$-Alkyl, bevorzugt Methyl, Ethyl, tert.-Butyl, oder für Trimethylsilylethyl steht, und

$R^1$ - die angegebene Bedeutung hat,

in einem ersten Schritt mit Aldehyden der Formel (XIII)

$$R^2\text{-CHO} \qquad \text{(XIII)}$$

in welcher

$R^2$ - die angegebene Bedeutung hat,

gegebenenfalls in inerten organischen Lösungsmitteln wie Alkoholen z.B. Methanol, Ethanol oder Propanol, gegebenenfalls in Anwesenheit von Piperidin und Eisessig zu den Ylidenverbindungen der allgemeinen Formel (XIV)

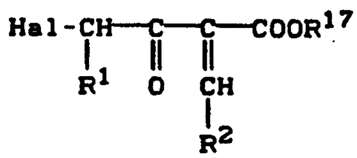

(XIV)

in welcher

$R^1$, $R^2$ und $R^{17}$ die angegebene Bedeutung haben, umsetzt, und in einem zweiten Schritt die Verbindungen (XIV) mit Acetylthioharnstoff in Wasser und/oder inerten organischen Lösungsmitteln wie Alkoholen, z.B. Methanol, Ethanol, Propanol, oder Formamiden wie z.B. Dimethylformamid, insbesondere in Mischungen aus Wasser und Dimethylformamid zu Acetylaminothiazolen der Formel (XV)

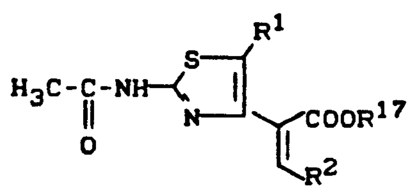

(XV)

in welcher

$R^1$, $R^2$ und $R^{17}$ die angegebene Bedeutung haben,

umsetzt,
und dann die Ester der Formel (XV) mit Hilfe von wäßrigen Basen wie beispielsweise Kaliumhydroxid, Natriumhydroxid, gegebenenfalls in Anwesenheit von Alkoholen wie Methanol oder Ethanol zu Säuren der Formel (IIa) hydrolysiert. Die Durchführung des Verfahrens erfolgt in Analogie zu dem in DE-OS 3 037 997 beschriebenen Verfahren.

Die Verbindungen (IIb), in welcher

$R^1$ - für Halogen, Alkoxy oder Alkylthio steht und

$R^2$ - die bereits angegebene Bedeutung hat,

erhält man, wenn man Verbindungen der Formel (XV), in denen $R^1$ = H ist, mit Halogenierungsmitteln wie beispielsweise Brom, Chlor, N-Bromsuccinimid, N-Chlorsuccinimid, Sulfonylchlorid oder Sulfurylbromid, insbesondere Sulfurylbromid und Sulfurylchlorid, gegebenenfalls in inerten organischen Lösungsmitteln wie Halogenkohlenwasserstoffen wie Methylenchlorid, Chloroform, Tetrachlorkohlenstoff, 1,2-Dichlorethan, 1,1,2-Trichlorethan, Dichlorethylen oder Kohlenwasserstoffen wie Benzol, Toluol, Xylol, insbesondere in Halogenkohlenwasserstoffen, halogeniert,
gegebenenfalls E/Z-Gemische (bezüglich der Doppelbindung) nach üblichen Verfahren z.B. durch Chromatographie oder Kristallisation trennt und gegebenenfalls Chlor oder Brom gegen Fluor, Alkoxy oder Alkylthio nach üblichen Methoden austauscht.

Die Durchführung dieses Verfahrens ist im Prinzip in DE-OS 2 758 000, 2 758 001 sowie EP-OS 34 760 und 55 465 beschrieben. Die Verbindungen (IIb) können in Analogie zu den dort beschriebenen Verfahren hergestellt werden.

Die als Ausgangsverbindungen eingesetzten Aldehyde der Formel (XII) sind bekannt oder können nach literaturbekannten Methoden hergestellt werden [E. Mosettig, Organic Reactions Vol. III, 218 ff. (1954)].

Die als Ausgangsverbindungen eingesetzten Halogenverbindungen der Formel (XIV) sind bekannt oder können nach bekannten Methoden hergestellt werden.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und

bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können zum Zwecke der Erweiterung des Wirkungsspektrums und um eine Wirkungssteigerung speziell bei β-Lactamasebildenden Bakterien zu erzielen mit anderen antimikrobiellen Wirkstoffen und Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

Herstellungsbeispiele

Beispiel 1

4-Brom-3-oxovaleriansäureethylester

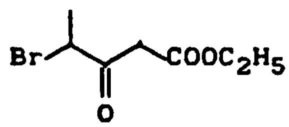

Zu einer Lösung von 250 g 3-Oxovaleriansäureethylester und 0,5 g p-Toluolsulfonsäure in 55 ml Methylchlorid werden bei Raumtemperatur unter Rühren 277 g Brom getropft. Man rührt eine Stunde bei 30° C nach, versetzt mit Eiswasser und trennt die organische Phase ab. Die wäßrige Phase wird noch einmal mit Methylenchlorid extrahiert und die vereinigten organischen Phasen dann über Magnesiumsulfat getrocknet und eingedampft. Das Produkt wird roh weiterverarbeitet.
Ausbeute: 350 g = 90 % der Theorie

Beispiel 2

2-Ethyliden-4-brom-3-oxovaleriansäureethylester

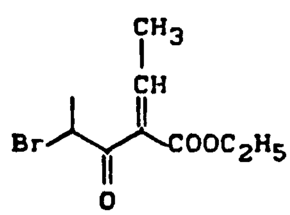

Eine Lösung von 100 g (0,45 Mol) 4-Brom-3-oxovaleriansäureethylester in 100 ml abs. Methylenchlorid wird unter Stickstoff auf -20° C abgekühlt und mit 50 g (1,12 Mol) Acetaldehyd versetzt. Anschließend wird 1 g Piperidin, gelöst in 10 ml Methylenchlorid, bei -20° C zugetropft.

Man rührt 30 Minuten bei -20° C, dann 2,5 Stunden bei -5° C nach und versetzt dann mit 100 ml eiskalter 2 N Salzsäure. Die organische Phase wird abgetrennt, die wäßrige Lösung noch einmal mit Methylenchlorid gewaschen und die vereinigten organischen Phasen werden getrocknet und eingedampft. Man erhält ein Rohprodukt, das laut Gaschromatogramm ein 2,5:1-Isomerengemisch in 88 %iger Reinheit ist und roh weiterverarbeitet werden kann.
Ausbeute: 95 g = 85 % der Theorie

Beispiel 3

Z-2-(2-Acetamido-5-methylthiazol-4-yl)but-2-ensäureethylester

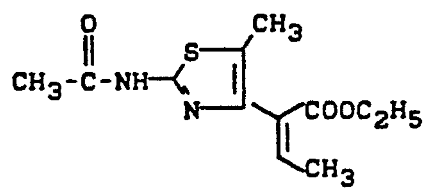

24,9 g (theoret. 0,1 Mol) 2-Ethyliden-4-brom-3-oxo-valeriansäureethylester (Rohprodukt) und 8,2 g (0,07 Mol) Acetylthioharnstoff werden in einem Gemisch aus 50 ml Wasser und 90 ml Dimethylformamid gelöst. Man erwärmt unter Rühren eine Stunde auf 85° C, kühlt dann eine Stunde im Eisbad ab und saugt das ausgefallene Produkt ab. Es wird einmal mit Wasser/DMF = 4/1, einmal mit Wasser/DMF = 9/1 und einmal mit Wasser gewaschen und getrocknet. Ausbeute: 10,7 g = 39,3 % der Theorie; Schmelzpunkt: 166° C.

Das Produkt ist nach HPLC (Säule RP8 10µ 250/4; Laufmittel Puffer pH 7/Acetonitril 40/60; 2 ml/min) zu 94 % isomerenrein, durch NMR-Vergleich mit bekannten Produkten und aufgrund der antibakteriellen Wirkung der Folgeprodukte wird ihm die Z-Konfiguration zugeordnet.

NMR*(DMSO-$d_6$): δ (ppm) = 6,32 [1] q, J=8 Hz, 4.20 [2] q, J=7 Hz, 2,24 [3] s, 2,12 [3] s, 1,97 [3] d, J=8 Hz, 1,23 [3] t, J=7 Hz.

## Beispiel 4

Z-2-(2-Amino-5-methylthiazol-4-yl)but-2-ensäure

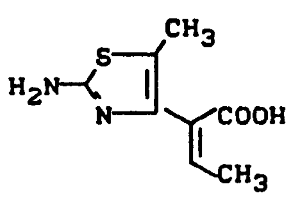

* NMR bedeutet in diesem und allen folgenden Beispielen $^1$H-NMR.

27 g (0,1 Mol) Z-2-(2-Acetamino-5-methylthiazol-4-yl)but-2-ensäureethylester werden auf einmal in eine bei 85°C gerührte Lösung von 29 g (0,5 Mol) Kaliumhydroxid in 198 ml Wasser gegeben. Man rührt 2 Stunden bei 80°C nach, kühlt dann mit einem Eisbad und stellt unter Eiskühlung mit konzentrierter Salzsäure den pH auf 3,0 ein. Die wäßrige Lösung wird vom schmierigen Rückstand dekantiert, auf ein Fünftel ihres Volumens eingeengt und erneut dekantiert. Die vereinigten Rückstände werden mit Aceton kristallisiert. Ausbeute 7,8 g = 39,1 % der Theorie.
HPLC (Säule $NH_2$ 5µ, 250/4, Laufmittel Methanol/Wasser/Eisessig 100/10/6, 1,5 ml/min): 1.39 min, Reinheit 96 %

NMR (DMSO-$d_6$): δ (ppm) = 6,80 [2] bs, 6,19 [1] q, J=8 Hz, 2,12 [3] s, 1,92 [3] d, J=8 Hz.

## Beispiel 5

**E/Z-2-(2-Acetamido-5-chlorthiazol-4-yl)but-2-ensäure-ethylester**

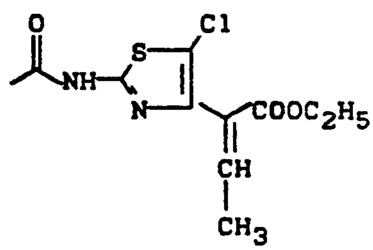

50,8 g (0,2 Mol) Z-2-(2-Acetamidothiazol-4-yl)buten-2-säureethylester werden bei -40°C in einem Gemisch aus 1,2 l Chloroform und 140 ml Eisessig suspendiert. Unter Rühren tropft man eine Lösung von 31 g (0,44 Mol) Chlor in Tetrachlorkohlenstoff zu, läßt dann auf Raumtemperatur kommen und dampft ein. Kristallisation aus Ether/Ligroin liefert 52 g = 72 % der Theorie, Schmelzpunkt: 140-142°C. Es entsteht ein 1:1 Diastereomerengemisch von 2-(2-Acetamido-5-chlorthiazol-4-yl)-2,3-dichlorbutansäureethylester.

NMR ($CDCl_3$) =

Isomer A: δ (ppm) = 7,56 [1] bs, 5,22 [1] q, J=8 Hz, 4,33 [2] q, J=7 Hz, 2,29 [3] s, 1,78 [3] d, J=8 Hz, 1,30 [3] t, J=7 Hz.

Isomer B: δ (ppm) = 7,64 [1] bs, 5,10 [1] q, J=8 Hz, 4,32 [2] q, J=7 Hz, 2,31 [3] s, 1,70 [3] d, J=8 Hz, 1,31 [3] t, J=7 Hz.

MS: 358 ($M^+$), 316 ($M^+$ - $CH_2$=C=O)

| | | | | | |
|---|---|---|---|---|---|
| Ber.: | C 36,7 | H 3,6 | N 7,8 | S 8,9 | Cl 29,6 |
| Gef.: | C 36,8 | H 3,7 | N 8,2 | S 8,8 | Cl 28,5 |

In eine Lösung von 5 g (13,9 Mol) dieses Produkts in 20 ml siedendem Methanol werden zuerst eine Spatelspitze Zinkchlorid und dann innerhalb von 30 Minuten portionsweise 1 g (15,3 Mol) Zinkstaub eingetragen. Man kocht noch eine Stunde nach, kühlt dann ab und dampft ein. Der Rückstand wird in Eiswasser/Essigester aufgenommen und die Lösung mit 6 n Salzsäure auf pH 2 eingestellt. Nach Abtrennen des

Wassers wird die organische Phase noch einmal mit 1 n Salzsäure, einmal mit Wasser, dreimal mit gesättigter Natriumbicarbonatlösung und einmal mit Wasser extrahiert, getrocknet und eingedampft. Chromatografie des Rohproduktes an Kieselgel (Laufmittel Toluol/Aceton 3/1) liefert 600 mg des reinen Z-Isomeren, Fp. 186-7° (Ether) und 500 mg E/Z-Gemisch. Gesamtausbeute 24,9 % der Theorie.

NMR ($CDCl_3$)

Z-Isomer: $\delta$ (ppm) = 10.27 [1] bs, 6.58 [1] q, J = 8 Hz, 4.23 [2] q, J = 7 Hz, 2.13 [3] s, 2.06 [3] d, J = 8 Hz, 1.22 [3] t, J = 7 Hz.

E-Isomer: $\delta$ (ppm) = 10.50 [1] bs, 7.26 [1] q, J = 8 Hz, 4.18 [2] q, J = 7 Hz, 2.11 [3] s, 1.72 [3] d, J = 8 Hz, 1.20 [3] t, J = 7 Hz.

## Beispiel 6

Z-2-(5-Chlor-2-bis-tert.-butoxycarbonylaminothiazol-4-yl)-but-2-ensäureethylester

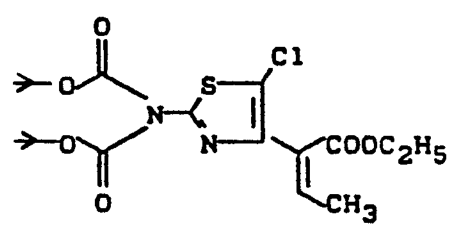

Zu einer Lösung von 25,5 g (0,09 Mol) des Z-2-(2-Acetamido-5-chlorthiazol-4-yl)but-2-ensäureethylester in 110 ml Pyridin werden 64,2 g (0,29 Mol) Di-tert.-butyldicarbonat unter Rühren bei Raumtemperatur zugetropft. Man rührt bei Raumtemperatur über Nacht, erwärmt eine Stunde auf 50°C und kontrolliert den Umsatz dünnschichtchromatographisch. Falls erforderlich werden noch einmal 10,7 g (0,05 Mol) Di-tert.-butyldicarbonat zugegeben und die Lösung eine weitere Nacht gerührt. Dann wird das Pyridin abgezogen, das verbleibende Öl in Essigester gelöst und die Lösung dreimal mit 6n Salzsäure und einmal mit Wasser gewaschen. Trocknen und Eindampfen ergibt ein dünnflüssiges Öl, das laut NMR aus 80 % Produkt und 20 % Di-tert.-butyldicarbonat besteht. Es kann roh in die nächste Stufe eingesetzt werden.
Ausbeute: 34 g.

NMR ($CDCl_3$): $\delta$ (ppm) = 6,62 [1] q, J=8 Hz, 4,25 [1] q, J=7 Hz, 2,02 [3] d, J=8 Hz, 1,53 [18] s, 1,27 [3] t, J=7 Hz.

Beispiel 7

E/Z-2-(5-Chlor-2-tert.-butoxycarbonylaminothiazol-4-yl)but-2-ensäure

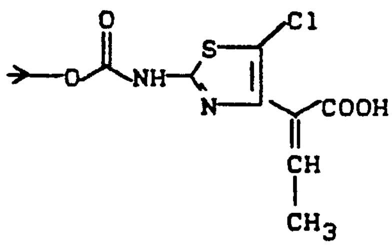

25 g (theoret. 0,056 Mol) Z-2-(5-Chlor-2-di-tert.-butyloxycarbonylaminothiazol-4-yl)but-2-ensäureethylester (Rohprodukt) gelöst in 200 ml Ethanol, werden bei Raumtemperatur zu einer Lösung von 22,8 g (0,56 Mol) Natriumhydroxid in 200 ml Wasser gegeben. Man rührt bei Raumtemperatur über Nacht, zieht das Ethanol ab und extrahiert die wäßrige Lösung dreimal mit Essigester. Die wäßrige Phase wird mit Essigester versetzt, mit 6 N Salzsäure auf pH 1 gestellt und insgesamt dreimal mit Essigester extrahiert. Trocknen und Eindampfen dieser letzten Essigesterphase ergibt ein Rohprodukt, aus dem mit Tetrachlorkohlenstoff 3,8 g des E-Isomeren kristallisiert werden können. Die Mutterlauge wird eingedampft, sie enthält laut HPLC (Säule $NH_2$, 5µ, 125/4, Laufmittel Methanol/Wasser/ Eisessig 100/10/3, 2 ml/min) 41 % E-Isomeres (Retentionszeit 2,4 min) und 22 % Z-Isomeres (Retentionszeit 6,0 min). Dieses Gemisch wird in Methanol gelöst und an Lewatit MP 62 unter Zusatz steigender Mengen Säure (1. Methanol, 2. 0,5 % Eisessig, 3. 1 % Eisessig, 4. 5 % konz. Salzsäure, 5. 10 % konz. Salzsäure) getrennt. Zuerst wird das E-Isomere gemeinsam mit allen Verunreinigungen eluiert, am Ende das reine Z-Isomere.

E-Isomer

Ausbeute: 3,8 g = 26,6 % der Theorie, Schmelzpunkt: >190° C ($CCl_4$)

NMR ($CDCl_3$): δ (ppm) = 8,72 [1] bs, 7,26 [1] q, J=8 Hz, 1,73 [3] d, J=8 Hz, 1,50 [9] s.

Z-Isomer

Ausbeute: 0,9 g = 6,3 % der Theorie, Schmelzpunkt >190° ($CCl_4$)

NMR ($CDCl_3$): δ (ppm) = 10,9 [1] bs, 6,63 [1] q, J=8 Hz, 2,16 [3] d, J=8 Hz, 1,56 [9] s.

Beispiel 8

Z-2-(2-Amino-5-chlorthiazol-4-yl)but-2-ensäure

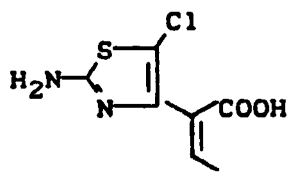

0,7 g Z-2-(5-Chlor-2-tert.-butoxycarbonylaminothiazol-4-yl)but-2-ensäure werden bei 0° C unter Stickstoff in 5 ml Trifluoressigsäure gelöst. Man rührt eine Stunde bei Raumtemperatur, dampft kalt ein und schlämmt den Rückstand mit Essigester auf. Unter Rühren wird mit gesättigter Natriumbicarbonatlösung auf pH 3,5 eingestellt, wobei das Produkt zuerst in Lösung geht, dann aber wieder ausfällt. Es wird abgesaugt, mit Essigester gewaschen und getrocknet.

Ausbeute: 0,3 g = 62,5 % der Theorie; Schmelzpunkt: sintert ab 130° C.

NMR ($DMSO-d_6$): δ (ppm) = 12,73 [1] bs, 7,18 [2] s, 6,40 [1] q, J=8 Hz, 1,92 [3] d, J=8 Hz.

Beispiel 9

4-Chlor-2-ethyliden-3-oxobuttersäure-tert.-butylester

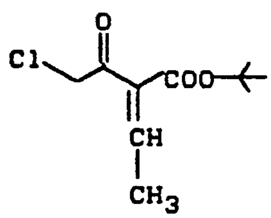

Zu einer gerührten Mischung aus 212 g (1,1 Mol) 4-Chlor-3-oxobuttersäure-tert.-butylester und 97 g (2,2 Mol) Acetaldehyd werden bei -20° C unter Stickstoff 1,5 ml Piperidin zugetropft. Man rührt 6 Stunden bei -20° C nach und arbeitet dann analog zu Beispiel 2 auf. Roh-Ausbeute 210 g, Gehalt nach GC: 6 % Ausgangsmaterial, 52 + 37 % Isomerengemisch des Produktes. Das Produkt wird roh weiterverarbeitet.

NMR ($CDCl_3$)

Isomer A: δ (ppm) = 7,11 [1] q, J=8 Hz, 4,33 [2] s, 1,91 [3] d, J=8 Hz, 1,49 [9] s.

Isomer B: δ (ppm) = 7,08 [1] q, J=8 Hz, 4,40 [2] s, 2,08 [3] d, J=8 Hz, 1,54 [9] s.

0223184

Beispiel 10

Z-2-(2-Acetamidothiazol-4-yl)but-2-ensäure-tert.-butylester

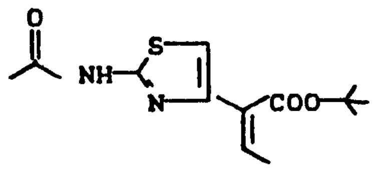

Das Rohprodukt eines 2,2-Mol-Ansatzes 4-Chlor-2-ethyliden-3-oxobuttersäure-tert.-butylester wird, gelöst in 100 ml DMF zu einer auf 85°C erwärmten Lösung von 236 g (2 Mol) Acetylthioharnstoff in einem Gemisch aus 360 ml Wasser und 720 ml DMF, gegeben. Man rührt 2 Stunden bei 85°C, engt ein, nimmt den Rückstand in Essigester auf und extrahiert die Lösung dreimal mit Wasser. Nach Trocknen und Einengen werden mit Ether Nebenprodukte ausgefällt.

Die vereinigten Mutterlaugen werden eingedampft. Aus dem Rückstand läßt sich ein Teil des gewünschten Produktes mit Ligroin/Ether kristallisieren (24,8 g), die Mutterlauge wird über Kieselgel chromatographiert (Laufmittel 1. Toluol, 2. Toluol/Aceton 9/1). Die Produkt enthaltenden Fraktionen werden ebenfalls mit Ligroin/Ether kristallisiert. Ausbeute: 109 g = 17,5 % der Theorie, Schmelzpunkt: 143-45°C.

NMR (DMSO-$d_6$): δ (ppm) = 12,2 [1] bs, 6,90 [1] s, 6,70 [1] q, J=8 Hz, 2,20 [3] s, 1,87 [3] d, J=8 Hz, 1,54 [9] s.

Beispiel 11

Z-2-(2-Acetamido-5-chlorthiazol-4-yl)but-2-ensäure-tert.-butylester

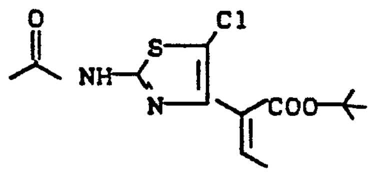

78 g (0,28 Mol) Z-2-(2-Acetamidothiazol-4-yl)but-2-ensäure-tert.-butylester werden in einem Gemisch aus 2 l Tetrachlorkohlenstoff und 15 ml Eisessig bei 0°C suspendiert. Unter Rühren tropft man innerhalb von 5 Stunden 43 g (0,32 Mol) Sulfurylchlorid, gelöst in 600 ml Tetrachlorkohlenstoff zu. Die Lösung wird einmal mit gesättigter Natriumbicarbonatlösung und einmal mit Wasser extrahiert, getrocknet und eingeengt auf etwa 200 ml. Das ausgefallene Produkt wird abgesaugt und mit Ether gewaschen (53,4 g). Die Mutterlauge wird vollständig eingedampft und durch Kristallisation des Rückstandes mit Ether/Ligroin wird eine zweite Fraktion des Produktes gewonnen (6 g). Ausbeute: 59,4 g = 67,8 % der Theorie.

NMR ($CDCl_3$): δ (ppm) = 10,21 [1] bs, 6,53 [1] q, J=8 Hz, 2,18 [3] s, 2,07 [3] d, J=8 Hz, 1,49 [9] s.

Beispiel 12

Z-2-(5-Chlor-2-tert.-butoxycarbonylaminothiazol-4-yl)but-2-ensäure-tert.-butylester und

Beispiel 13

Z-2-(5-chlor-2-bis-tert.-butoxycarbonylaminothiazol-4-yl)-but-2-ensäure-tert.-butylester

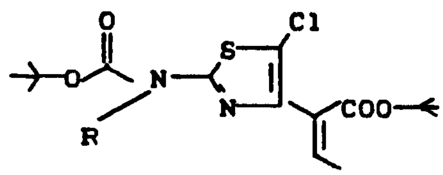

| Bsp. | R |
|---|---|
| 12 | H |
| 13 | $(H_3C)_3COCO$ |

Eine Lösung von 59 g (0,19 Mol) Z-2-(2-Acetamido-5-chlor-thiazol-4-yl)but-2-ensäure-tert.-butylester und 122 g (0,56 Mol) Di-tert.-butyldicarbonat in 500 ml Pyridin wird 3 Tage bei Raumtemperatur gerührt und dann eingeengt. Den Rückstand nimmt man in Essigester auf, extrahiert dreimal mit 20 %iger Salzsäure und einmal mit gesättigter Kochsalzlösung. Nach Trocknen über Natriumsulfat und Eindampfen wird an 3 kg Kieselgel chromatographiert (Laufmittel 1. Toluol, 2. Toluol/Essigester 9/1). Es wird zuerst Beispiel 13 dann 12 eluiert.

Beispiel 13:

Ausbeute: 25 g Öl (= 28,2 % der Theorie) enthält noch Reste $/[(H_3C)_3C-OCO]_2O$

NMR ($CDCl_3$) δ (ppm) = 6,60 [1] q, J=8 Hz, 2,57 [3] d, J=8 Hz, 1,55 [18] s, 1,54 [9] s.

Beispiel 12:

Ausbeute: 40 g Hartschaum = 57,3 % der Theorie

NMR ($CDCl_3$): δ (ppm) = 9,08 [1] bs, 6,54 [1] q, J=8 Hz, 2,07 [3] d, J=8 Hz, 1,52 [18] s.

Beispiel 14

Z-2-(2-Amino-5-chlorthiazol-4-yl)but-2-ensäure

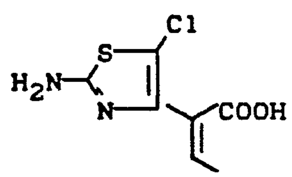

40 g Z-2-(5-Chlor-2-tert.-butoxycarbonylaminothiazol-4-yl)but-2-ensäure werden bei 0°C in 200 ml Trifluoressigsäure gelöst. Nach 2-stündigem Stehen bei Raumtemperatur wird die Lösung kalt eingedampft und der Rückstand mit Wasser kristallisiert, abgesaugt und getrocknet.

Ausbeute: 30 g Trifluoressigsäuresalz des Produktes = 87,6 % der Theorie

NMR (DMSO-$d_6$): δ (ppm) = 6,52 [1] q, J=8 Hz, 2,00 [3] d, J=8 Hz.

Dieses Produkt wird analog Beispiel 8 in die freie Base überführt, deren analytische Daten mit den in Beispiel 8 angegebenen übereinstimmen.
Ausbeute: 18,8 g = 80,5 % der Theorie.

Beispiel 15

Z-7-/Z-(2-Amino-5-methylthiazol-4-yl)but-2-enoyl7amino-3-pyridinium-methyl-cefem-4-carboxylat

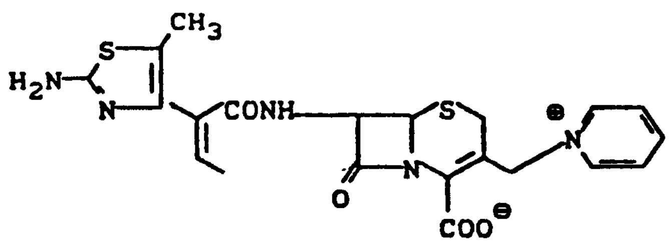

Eine Lösung von 1,2 g Z-2-(2-Amino-5-methylthiazol-4-yl)but-2-ensäure (6 mmol) und 1,8 ml Triethylamin (12,8 mmol) in 7,2 ml DMF wird unter Stickstoff auf -50°C gekühlt und mit 0,5 ml (6,3 mmol) Methansulfonsäurechlorid versetzt. Man rührt 2 Stunden bei -50°C nach. Zu der -50°C kalten Lösung gibt man eine Lösung von 1,7 g (4,9 mmol) 7-Amino-3-pyridiniummethyl-3-cefem-4-carboxylat-hydrochlorid-hydrat und 2,3 ml (16 mmol) Triethylamin in 2,3 ml Wasser, entfernt das Kühlbad, rührt 10 Minuten nach und

gießt die Lösung dann auf 70 ml Aceton. Das ausgefallene Produkt wird abgesaugt und getrocknet. Chromatographie an LOBAR C (RP 8, Laufmittel Wasser/Acetonitril 8/2) liefert 0,3 g reines Produkt = 10,5 % der Theorie.

NMR ($DMSO-d_6$): δ (ppm) = 9,49 [2] d, J=6 Hz, 9,10 [1] d, J=7 Hz, 8,62 [1] bt, J=7 Hz, 8,20 [2] bt, J=7 Hz, 6,75 [2] bs, 6,12 [1] q, j=8 Hz, 5,72 [1] dd, J=7 Hz, J=5 Hz, 5,68 [1] d, J=13 Hz, 5,13 [1] d, J=13 Hz, 5,10 [1] d, J=5 Hz, 3,54 [1] d, J=18 Hz, 3,06 [1] d, J=18 Hz, 2,08 [3] s, 1,80 [3] d, J=8 Hz.

Beispiel 16

Natrium-Z-7-/2-(2-amino-5-chlorthiazol-4-yl)but-2-enoyl7 amino-3-acetoxymethyl-3-cefem-4-carboxylat

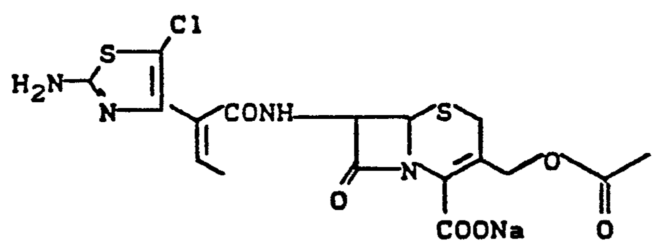

1,4 g Z-2-(2-Amino-5-chlorthiazol-4-yl)but-2-ensäure werden nach der Vorschrift von Beispiel 15 aktiviert und mit 2,09 g 7-ACS unter Zusatz von 1,9 g Triethylamin gekuppelt. Das Rohprodukt reinigt man über Diaion HP 20. Ausbeute: 1 g = 32 % der Theorie

NMR (DMSO-$d_6$): δ (ppm) = 9,19 [1] d, J=8 Hz, 7,29 [2] bs, 6,43 [1] q, J=8 Hz, 5,64 [1] dd, J=8 Hz, J=5 Hz, 5,07 [1] d, J=5 Hz, 5,03 [1] d, J=13 Hz, 4,79 [1] d, J=13 Hz, 3,51 [1] d, J=18 Hz, 3,24 [1] d, J=18 Hz, 2,03 [3] s, 1,89 [1] d, J=8 Hz.

Beispiel 17

Z-7-[2-(2-Amino-5-chlorthiazol-4-yl)but-2-enoyl]amino-3-pyridinium-methyl-3-cefem-4-carboxylat

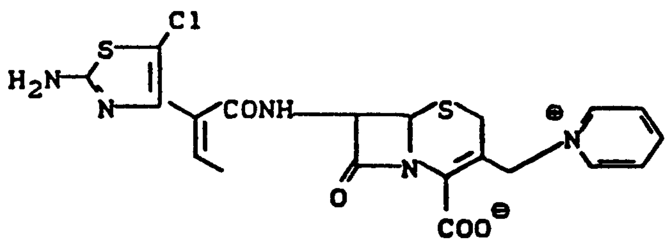

5,6 g (25,6 mmol) Z-2-(2-Amino-5-chlorthiazol-4-yl)but-2-ensäure werden nach der Vorschrift von Beispiel 15 aktiviert und dann mit einer Lösung von 6,8 g (19,7 mmol) 7-Amino-3-pyridiniummethyl-3-cefem-4-carboxylat-hydrochlorid-hydrat und 6,6 ml (47,4 mmol) Triethylamin in 20 ml Wasser gekuppelt. Reinigung des aus Aceton gefällten Rohproduktes an Diaion HP 20 liefert 6,5 g = 51,6 % der Theorie.

NMR (DMSO-$d_6$): δ (ppm) = 9,55 [2] d, J=6 Hz, 9,19 [1] d, J=7 Hz, 8,64 [1] bt, J=7 Hz, 8,20 [2] bt, J=6 Hz, 7,23 [2] bs, 6,36 [1] q, J=8 Hz, 5,71 [1] d, J=13 Hz, 5,69 [1] dd, J=7 Hz, J=5 Hz, 5,14 [1] d, J=13 Hz, 5,10 [1] d, J=5 Hz, 3,55 [1] d, J=18 Hz, 3,05 [1] d, J=18 Hz, 1,80 [3] d, J=8 Hz.

| | | | | | |
|---|---|---|---|---|---|
| ber. x 1,5 $H_2O$ = | C 46,3 | H 4,1 | N 13,5 | S 12,3 | Cl 6,8 |
| gef. = | C 46,3 | H 4,0 | N 13,4 | S 11,6 | Cl 7,1 |

Beispiel 18

Z-7-/2-(2-Amino-5-chlorthiazol-4-yl)but-2-enoyl7amino-3-(4-amino-carbonyl-1-methylpiperazinium)methyl-3-cefem-4-carboxylat

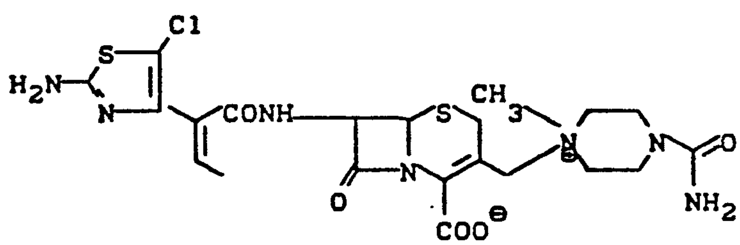

a.) 7-Amino-3-(4-aminocarbonyl-1-methylpiperazinium)methyl-3-cefem-4-carboxylat

Unter Stickstoff werden 9,36 g (24 mmol) 3-Acetoxymethyl-7β-phenylacetamido-3-cefem-4-carbonsäure bei Raumtemperatur in 100 ml absolutem Methylenchlorid aufgeschlämmt und durch Zugabe von 15,2 ml (72 mmol) N-Methyl-N-trimethylsilyltrifluoracetamid (MSTFA) in Lösung gebracht. Nach Abkühlen auf 0°C werden 14 ml (96 mmol) Trimethylsilyljodid zugegeben und die Reaktionslösung wird 1 h bei 0°C gerührt. Anschließend werden 34 g 1-Aminocarbonyl-4-methylpiperazin, gelöst in 150 ml DMF, zugegeben und die Lösung wird 30 Minuten nachgerührt. Dann werden 4,8 ml $H_2O$ zugegeben und nach weiteren 5 Minuten wird auf 400 ml Ether gegossen. Der Ether wird vom öligen Rückstand abdekantiert. 5 g des Produktes werden in 100 ml $H_2O$ gelöst und mit 4 n Triethylamin in Ethanol wird pH 7,8 eingestellt. Anschließend werden 6 g Penicillin-G-Acylase zugegeben und der pH wird durch Zugabe von Triethylamin konstant gehalten. Nach Beendigung der enzymatischen Spaltung wird von der Acylase abfiltriert und das Filtrat mit konzentrierter Salzsäure auf pH 2 eingestellt. Vom entstandenen Niederschlag wird über Kieselgur abgesaugt und das Filtrat wird zu 2 l Aceton getropft. Das gewünschte Produkt fällt als Hydrochlorid aus und wird abgesaugt und getrocknet.
Ausbeute: 3,1 g.

NMR ($D_2O$)
δ (ppm) = 5,39 [1] d, J=5 Hz, 5,16 [1) d, J=5 Hz, 4,80 [1] d, J=13 Hz, 4,14 [1] d, J=13 Hz, 3,85 - 4,02 [3] m, 3,35 - 3,72 [7] m, 3,22 [3) s.

b.) Z-7-[2-(2-Amino-5-chlorthiazol-4-yl)but-2-enoyl]amino-3-(4-aminocarbonyl-1-methylpiperazinium)methyl-3-cefem-4-carboxylat

0,56 g (2,56 mmol) Z-2-(2-Amino-5-chlorthiazol-4-yl)but-2-ensäure werden analog zu Beispiel 17 mit 0,8 g 7-Amino-3-(4-aminocarbonyl-1-methylpiperazinium)methyl-3-cefem-4-carboxylat-hydrochlorid gekuppelt.
Ausbeute nach Chromatographie an Diaion HP 20: 0,5 g

NMR (DMSO-$d_6$): δ (ppm) = 9,24 [1] d, J=8 Hz, 7,25 [2) bs, 6,40 [1] q, J=8 Hz, 6,32 [2) bs, 5,66 [1] dd, J=8 Hz, J=5 Hz, 5,16 [1) d, J=5 Hz, 5,12 [1] bd, J=13 Hz, 4,01 [1] bd, J=13 Hz, 3,80 [3] m, 3,18 - 3,65 [7] m, 3,02 [3] s, 1,86 [3] d, J=8 Hz.

Beispiel 19

Z-7-/Z-(2-Amino-5-chlorthiazol-4-yl)but-2-enoyl7amino-3-(1-methyl-pyrrolidinium)methyl-3-cefem-4-carboxylat

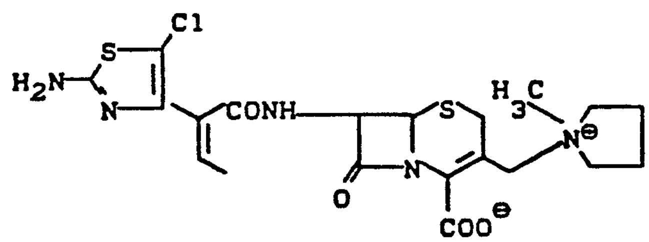

0,5 g (2,29 mmol) Z-2-(2-Amino-5-chlorthiazol-4-yl)but-2-ensäure werden nach der Vorschrift von Beispiel 15 aktiviert und dann mit einer Lösung von 0,65 g (1,85 mmol) 7-Amino-3-(1-methylpyrrolidinium)methyl-3-cefem-4-carboxylat und 0,6 ml Triethylamin in 1,5 ml Wasser gekuppelt.
Fällung aus Aceton liefert 0,68 g = 73,9 % der Theorie.

NMR (DMSO-$d_6$): δ (ppm) = 9,25 [1] d, J=7 Hz, 7,20 [2] bs, 6,37 [1] q, J=8 Hz, 5,79 [1] dd, J=7 Hz, J=5 Hz, 6,23 [1] d, J=5 Hz, 4,74 [1] d, J=13 Hz, 4,15 [1] d, J=13 Hz, 3,92 [1] d, J=18 Hz, 3,35-3,67 [5] m, 2,91 [3] s, 2,05 [4] m, 1,83 [3] d, J=8 Hz.

Beispiel 20

Z-7-/2-(2-Amino-5-chlorthiazol-4-yl)but-2-enoyl/amino-3-cefem-4-carbonsäurebenzhydrylester

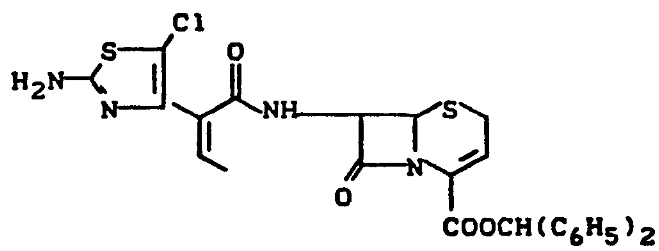

480 mg Z-2-(2-Amino-5-chlorthiazol-4-yl)but-2-ensäure werden nach der Vorschrift von Beispiel 15 aktiviert. Zu der -50°C kalten Lösung gibt man eine Lösung von 732 mg 7β-Amino-3-cefem-4-carbonsäurebenzhydrylester in 5 ml absol. Methylenchlorid und 360 µl Diisopropylethylamin. Man entfernt das Kühlbad und rührt 1 h bei Raumtemperatur nach. Der gesamte Ansatz wird auf Wasser gegeben und mehrmals mit Methylenchlorid extrahiert. Die vereinigten organischen Extrakte werden mit gesättigter $NaHCO_3$-Lösung und Wasser gewaschen, über Magnesiumsulfat getrocknet und dann über Kieselgel chromatographiert (Laufmittel Toluol/Aceton 8/2).

Ausbeute: 330 mg (27 %)

NMR (DMSO-$d_6$): δ (ppm) = 9,32 [1] d, J = 9 Hz, 7,20-7,60 [10] m, 6,94 [1] s, 6,78 [1] dd, J = 3 Hz, J = 5 Hz, 6,42 [1] q, J = 8 Hz, 5,92 [1] dd, J = 9 Hz, J = 5 Hz, 5,18 [1] d, J = 5 Hz, 3,68 [2] m, 1,87 [3] d, J = 8 Hz.

Beispiel 21

Natrium-Z-7-/2-(2-amino-5-chlorthiazol-4-yl)but-2-enoyl7amino-3-cefem-4-carboxylat

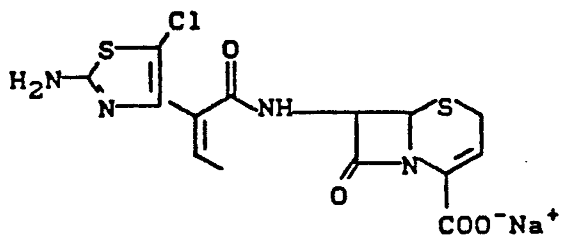

210 mg Z-7-/2-(2-Amino-5-chlorthiazol-4-yl)but-2-enoyl7amino-3-cefem-4-carbonsäurebenzylhydrylester werden in 3 ml absol. Methylenchlorid gelöst. Nach Zugabe von 1,3 ml Anisol und 1,3 ml Trifluoressigsäure wird 25 min bei 0°C gerührt. Anschließend wird im Vakuum eingeengt, der ölige Rückstand wird mit n-Hexan verrührt, abgesaugt und mit $NaHCO_3$ in Wasser gelöst. Die wäßrige Lösung wird über Diaion HP 20 gereinigt.

Ausbeute 94 mg (60 %)

NMR (DMSO-$d_6$): δ(ppm) = 9,11 [1] d, J = 9 Hz, 7,20 [2] bs, 6,38 [1] q, J = 8 Hz, 5,98 [1] m, 5,64 [1] dd, J = 5 Hz, J = 9 Hz, 4,97 [1] d, J = 5 Hz, 3,51 [1] dd, J = 2 Hz, J = 18 Hz, 3,30 [1] m, 1,85 [3] d, J = 8 Hz.

Patentansprüche

1. Cephalosporine der allgemeinen Formel (I)

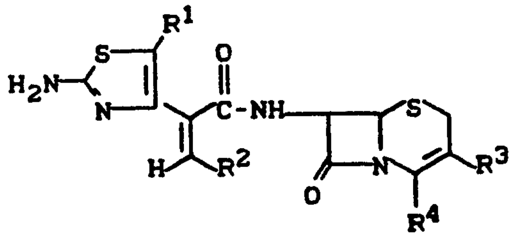

(I),

in welcher

$R^1$ - für Alkyl, Alkenyl, Alkoxy, Alkylthio, Halogen, Trifluormethyl oder Trifluormethoxy steht,

$R^2$ - für Wasserstoff, Alkenyl, für gegebenenfalls substituiertes Alkyl, gegebenenfalls substituiertes Aryl, gegebenenfalls substituiertes Heterocyclyl, Alkoxy, Carboxy, Alkoxycarbonyl, Halogen oder für eine Gruppe der Formel $-S(O)_n-B-A$ steht,

worin

n - 0, 1 oder 2 bedeutet,

B - eine direkte Bindung, Sauerstoff oder eine Gruppe -N-X darstellt, und

A und X unabhängig voneinander für Wasserstoff, gegebenenfalls substituiertes Alkyl, gegebenenfalls substituiertes Aryl oder gegebenenfalls substituiertes Heterocyclyl stehen, oder gemeinsam einen gegebenenfalls substituierten carbocyclischen oder heterocyclischen Ring bilden,

$R^3$ - für Wasserstoff, Alkyl, Alkenyl, Alkoxy, Alkylthio, Halogen oder für eine Gruppe der Formel $-CH_2-Y$ steht,

wobei

Y - für Hydroxy, Formyloxy, Alkanoyloxy, Aminocarbonyloxy, für gegebenenfalls substituiertes Heterocyclylthio, oder für einen stickstoffhaltigen und über N gebundenen positiv geladenen, bis zu insgesamt 3 Stickstoffatome enthaltenden 6-Ring steht, an den bis zu zwei weitere Ringe ankondensiert sein können und der gegebenenfalls substituiert sein kann, oder für eine Gruppe der Formel

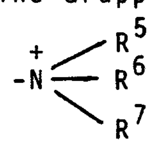

,

steht, worin

$R^5$,$R^6$ und $R^7$ gleich oder verschieden sein können und einen substituierten oder unsubstituierten Alkylrest oder einen mono- oder bicyclischen, gegebenenfalls substituierten, carbo- oder heterocyclischen Ring bedeuten oder

$R^5$ einen gegebenenfalls substituierten Alkylrest oder einen mono- oder bicyclischen, substituierten oder unsubstituierten, carbo- oder heterocyclischen Ring darstellt und

$R^6$ und $R^7$ gemeinsam mit dem N-Atom einen gegebenenfalls substituierten mono-oder polycyclischen Ring bilden, der gesättigt oder ungesättigt sein kann und Sauerstoff, Schwefel und/oder Stickstoff als weitere Heteroatome enthalten kann,

und

$R^4$ - je nach der Bedeutung von $R^3$ für $COO^-$ oder COOH steht,

und deren Salze.

2. Cephalosporine der Formel (I) gemäß Anspruch 1, in der

$R^1$, $R^2$ und $R^4$ die in Anspruch 1 angegebene Bedeutung haben und

$R^3$ - für Wasserstoff, Alkyl, Alkenyl, Alkoxy, Alkylthio, Halogen oder für eine Gruppe der Formel $-CH_2-Y$ steht, wobei

Y - für Hydroxy, Formyloxy, Alkanoyloxy, Aminocarbonyloxy, für gegebenenfalls substituiertes Heterocyclylthio, oder

- für einen Pyridiniumrest

(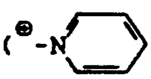)

steht, der gegebenenfalls ein- bis mehrfach gleich oder verschieden substituiert sein kann und an den ein oder zwei gegebenenfalls substituierte 3- bis 7-gliedrige Ringe ankondensiert sein können, die jeweils bis zu zwei Heteroatome und bis zu zwei Doppelbindungen enthalten können und auch aromatisch oder heteroaromatisch sein können, oder

- für einen Rest der Formel

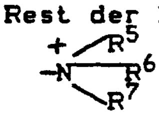

steht, wobei

$R^5$, $R^6$ und $R^7$ gleich oder verschieden voneinander sind und einen gegebenenfalls substituierten $C_1$-$C_6$-Alkylrest oder einen 3-bis 7-gliedrigen gegebenenfalls substituierten Ring darstellen, oder

$R^5$ die obige Bedeutung hat und

$R^6$ und $R^7$ gemeinsam mit dem N-Atom einen 3-bis 7-gliedrigen Ring bilden, der gesättigt oder ungesättigt sein kann und ein- oder mehrfach, bevorzugt 1- bis 3-fach gleich oder verschieden substituiert sein kann und ein oder zwei weitere Heteroatome enthalten kann, die Sauerstoff, Stickstoff und/oder Schwefel sein können.

3. Cephalosporine nach den Ansprüchen 1 bis 2 in der Z-Konfiguration.

4. Cephalosporine nach den Ansprüchen 1 bis 2 in der E-Konfiguration.

5. Cephalosporine der Formeln

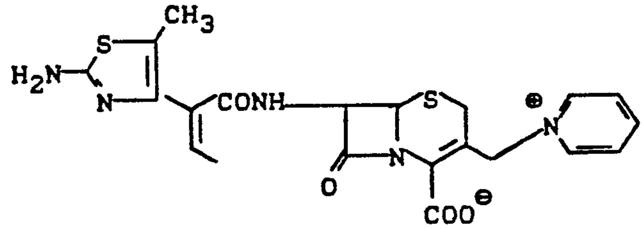

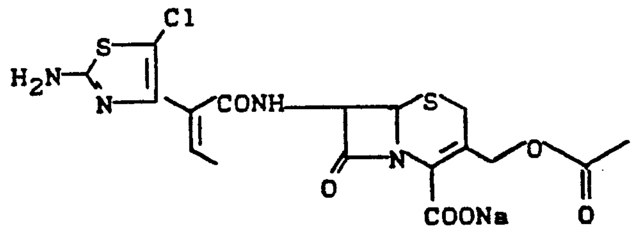

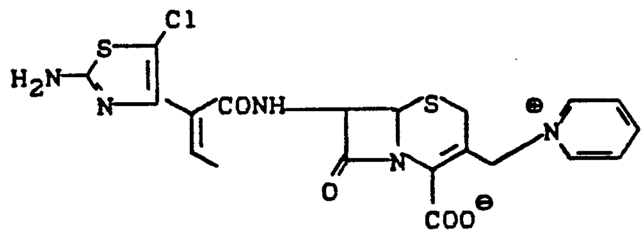

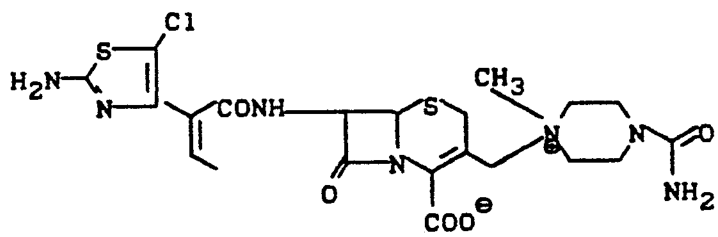

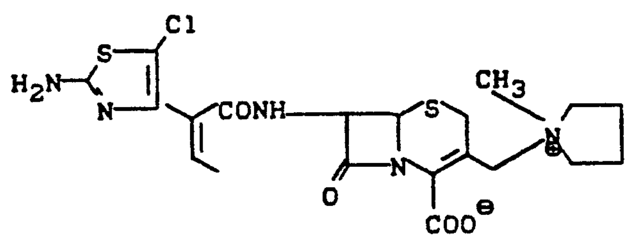

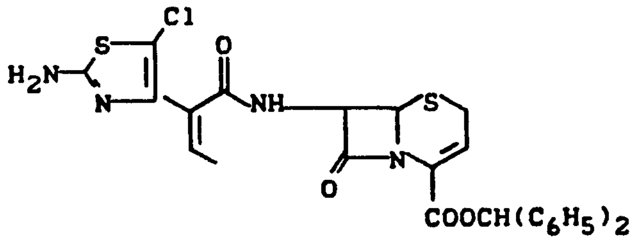

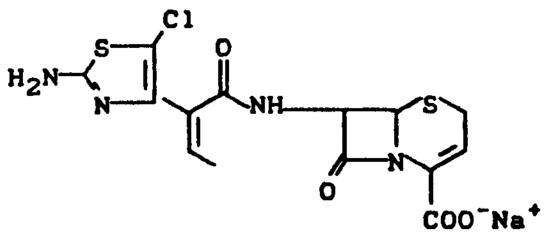

6. Cephalosporine der Formel (I) gemäß Anspruch 1,

in welcher

$R^1$ - für Methyl, Fluor, Chlor oder Trifluormethyl steht,

$R^2$ - für Methyl, Ethyl, Propyl, Isopropyl, Trifluormethyl, Carboxymethyl, Methoxycarbonylmethyl, Cyclopentyl, Cyclohexyl steht, oder

- für Phenyl, Chlorphenyl, Carboxyphenyl,
- für Pyridyl oder Aminothiazolyl,
- für Carboxy,
- für Methoxy, Ethoxy, Methylthio, Methylsulfonyl, Ethylsulfonyl, Phenylsulfonyl, Fluor oder Chlor steht,

$R^3$ - für Wasserstoff, Chlor, Fluor, Methyl, Methoxy, Methylthio, Trifluormethyl, Vinyl oder für die Gruppe der Formel $-CH_2-Y$ steht,

worin

Y - für Methoxy, Methylcarbonyloxy, Carbamoyloxy oder einen Rest der Formel

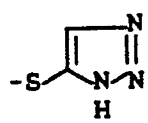 , 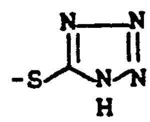 , 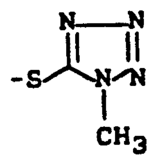 , 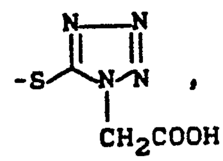 ,

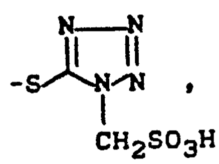 , 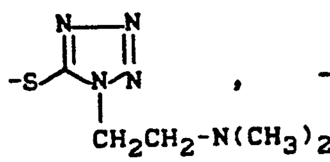 , 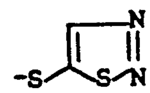 ,

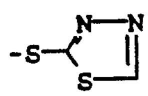 , 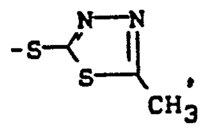 , 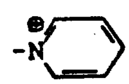 , 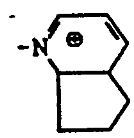 ,

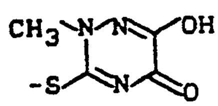 , 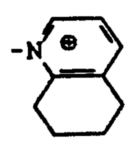 , 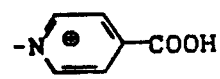 , 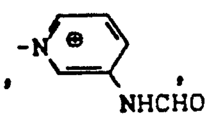 ,

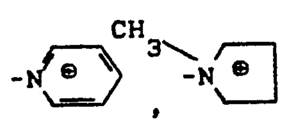 , 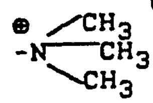 , 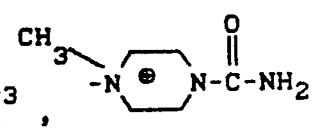 ,

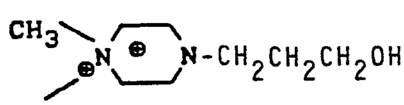 steht,

und

$R^4$ - für COOH oder $COO^-$ steht, und deren Salze.

7. Verfahren zur Herstellung von Cephalosporinen der allgemeinen Formel (I)

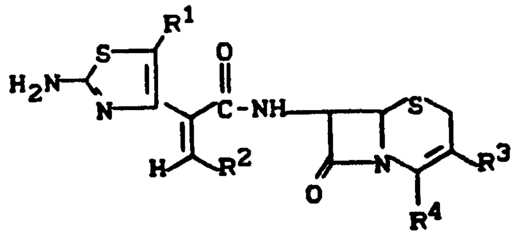

(I),

in welcher

$R^1$ - für Alkyl, Alkenyl, Alkoxy, Alkylthio, Halogen, Trifluormethyl oder Trifluormethoxy steht,

$R^2$ - für Wasserstoff, Alkenyl, für gegebenenfalls substituiertes Alkyl, gegebenenfalls substituiertes Aryl, gegebenenfalls substituiertes Heterocyclyl, Alkoxy, Carboxy, Alkoxycarbonyl, Halogen oder für eine Gruppe der Formel $-S(O)_n-B-A$ steht,

worin

n - 0, 1 oder 2 bedeutet,

B - eine direkte Bindung, Sauerstoff oder eine Gruppe -N-X darstellt, und

A und X unabhängig voneinander für Wasserstoff, gegebenenfalls substituiertes Alkyl, gegebenenfalls substituiertes Aryl oder gegebenenfalls substituiertes Heterocyclyl stehen, oder gemeinsam einen gegebenenfalls substituierten carbocyclischen oder heterocyclischen Ring bilden,

$R^3$ - für Wasserstoff, Alkyl, Alkenyl, Alkoxy, Alkylthio, Halogen oder für eine Gruppe der Formel $-CH_2-Y$ steht,

wobei

Y - für Hydroxy, Formyloxy, Alkanoyloxy, Aminocarbonyloxy, für gegebenenfalls substituiertes Heterocyclylthio, oder für einen stickstoffhaltigen und über N gebundenen positiven geladenen, bis zu insgesamt 3 Stickstoffatome enthaltenden 6-Ring steht, an den bis zu zwei weitere Ringe ankondensiert sein können und der gegebenenfalls substituiert sein kann, oder für eine Gruppe der Formel

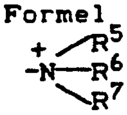

steht, worin

$R^5$,$R^6$ und $R^7$ gleich oder verschieden sein können und einen substituierten oder unsubstituierten Alkylrest oder einen mono- oder bicyclischen, gegebenenfalls substituierten, carbo- oder heterocyclischen Ring bedeuten oder

$R^5$ einen gegebenenfalls substituierten Alkylrest oder einen mono- oder bicyclischen, substituierten oder unsubstituierten, carbo- oder heterocyclischen Ring darstellt und

$R^6$ und $R^7$ gemeinsam mit dem N-Atom einen gegebenenfalls substituierten mono-oder polycyclischen Ring bilden, der gesättigt oder ungesättigt sein kann und Sauerstoff, Schwefel und Stickstoff als weitere Heteroatome enthalten kann

und

$R^4$ - je nach der Bedeutung von $R^3$ für $COO^-$ oder COOH steht,

und deren Salzen,

dadurch gekennzeichnet, daß Verbindungen der allgemeinen Formel (II)

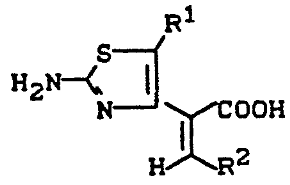

(II),

in welcher

$R^1$ und $R^2$ die angegebene Bedeutung haben,

in denen die Aminogruppe geschützt oder ungeschützt vorliegen kann, nach Aktivierung der Carboxylgruppe durch Überführung in ein gemischtes Anhydrid, nach Überführung in das Säurehalogenid oder nach Überführung in einen aktivierten Ester, mit Verbindungen der allgemeinen Formel (III),

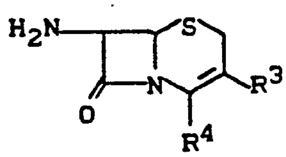

(III),

in welcher

$R^3$ und $R^4$ die oben angegebene Bedeutung haben,

zur Umsetzung gebracht werden, dann gegebenenfalls Schutzgruppen abgespalten werden und die gewünschten Salze oder aus Salzen die freien Säuren hergestellt werden.

8. Verfahren nach Anspruch 7, dadurch gekennzeichnet, daß man die Carbonsäuren der allgemeinen Formel (II) ohne Amino-Schutzgruppe aktiviert und dann mit den β-Lactamen der Formel (III), die als Salze mit einem Amin in Lösung gebracht wurden, kuppelt.

9. Verfahren nach Anspruch 7, dadurch gekennzeichnet, daß man die Verbindungen der Formel (1) gemäß folgendem Schema mit Sulfonsäurederivaten der Formel (IV) zu Anhydriden der Formel (V)

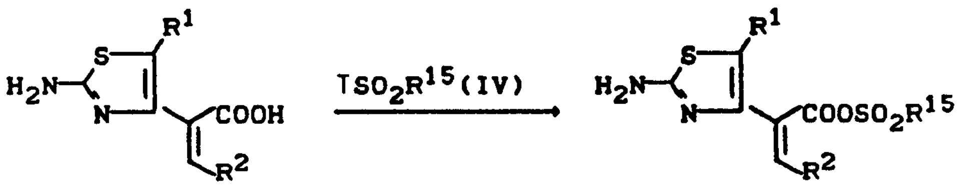


(II) (V)

aktiviert, worin

T - einen Rest $R^{15}$-$SO_2O$ oder Halogen darstellt und

$R^{15}$- einen Alkylrest mit 1 bis 10 C-Atomen bedeutet, der gegebenenfalls durch Fluor, Chlor, Cyano, Phenyl, Alkyloxycarbonyl, Alkoxy oder Alkyl substituiert sein kann, wobei letztere Alkylreste 1 bis 4 C-Atome tragen können, oder einen Phenylrest bedeutet, der gegebenenfalls durch Fluor, Chlor, Brom, Cyano, Alkyl, Alkoxy, Alkylthio, Alkylcarbonyl - wobei letztere Alkylgruppen 1 bis 4 C-Atome tragen können -Nitro, Trifluormethyl und Phenyl substituiert sein kann.

10. Verfahren nach Anspruch 9, dadurch gekennzeichnet, daß $R^{15}$ einen Methyl- oder p-Tolylrest darstellt.

11. Verfahren nach Anspruch 7, dadurch gekennzeichnet, daß man die Carbonsäuren der Formel (II) vor der Umsetzung mit den Verbindungen der Formel (III) mit N-Hydroxysuccinimid und Dicyclohexylcarbodiimid oder 1-Hydroxylbenztriazol und Dicyclohexylcarbodiimid aktiviert.

12. Verfahren nach Anspruch 7, dadurch gekennzeichnet, daß man die Carbonsäuren der Formel (II) mit Dicyclohexylcarbodiimid und 1-Hydroxybenztriazol in Dimethylformamid bei Raumtemperatur 2 bis 6 Stunden aktiviert, dann vom ausgefallenen Dicyclohexylharnstoff absaugt und mit einer Verbindung der Formel (III) in Form einer Lösung ihres Aminsalzes innerhalb von 2 bis 24 Stunden umsetzt.

13. Cephalosporine der allgemeinen Formel (I)

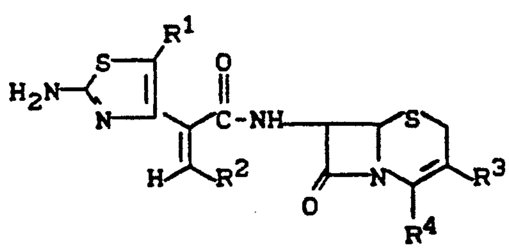

(I),

in welcher

$R^1$ - für Alkyl, Alkenyl, Alkoxy, Alkylthio, Halogen, Trifluormethyl oder Trifluormethoxy steht,

$R^2$ - für Wasserstoff, Alkenyl, für gegebenenfalls substituiertes Alkyl, gegebenenfalls substituiertes Aryl, gegebenenfalls substituiertes Heterocyclyl, Alkoxy, Carboxy, Alkoxycarbonyl, Halogen oder für eine Gruppe der Formel $-S(O)_n-B-A$ steht,

worin

n - 0, 1 oder 2 bedeutet,

B - eine direkte Bindung, Sauerstoff oder eine Gruppe -N-X darstellt, und

A und X unabhängig voneinander für Wasserstoff, gegebenenfalls substituiertes Alkyl, gegebenenfalls substituiertes Aryl oder gegebenenfalls substituiertes Heterocyclyl stehen, oder gemeinsam einen gegebenenfalls substituierten carbocyclischen oder heterocyclischen Ring bilden,

$R^3$ - für Wasserstoff, Alkyl, Alkenyl, Alkoxy, Alkylthio, Halogen oder für eine Gruppe der Formel $-CH_2-Y$ steht,

wobei

Y - für Hydroxy, Formyloxy, Alkanoyloxy, Aminocarbonyloxy, für gegebenenfalls substituiertes Heterocyclylthio, oder für einen stickstoffhaltigen und über N gebundenen positiven geladenen, bis zu insgesamt 3 Stickstoffatome enthaltenden 6-Ring steht, an den bis zu zwei weitere Ringe ankondensiert sein können und der gegebenenfalls substituiert sein kann, oder für eine Gruppe der Formel

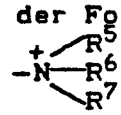

steht, worin

$R^5$,$R^6$ und $R^7$ gleich oder verschieden sein können und einen substituierten oder unsubstituierten Alkylrest oder einen mono- oder bicyclischen, gegebenenfalls substituierten, carbo- oder heterocyclischen Ring bedeuten oder

$R^5$ einen gegebenenfalls substituierten Alkylrest oder einen mono- oder bicyclischen, substituierten oder unsubstituierten, carbo- oder heterocyclischen Ring darstellt und

$R^6$ und $R^7$ gemeinsam mit dem N-Atom einen gegebenenfalls substituierten mono-oder polycyclischen Ring bilden, der gesättigt oder ungesättigt sein kann und Sauerstoff, Schwefel und Stickstoff als weitere Heteroatome enthalten kann

und

$R^4$ - je nach der Bedeutung von $R^3$ für $COO^-$ oder COOH steht,

und deren Salze zur Anwendung in einem Verfahren zur therapeutischen Behandlung des menschlichen oder tierischen Körpers.

14. Arzneimittel enthaltend Cephalosporine der allgemeinen Formel (I)

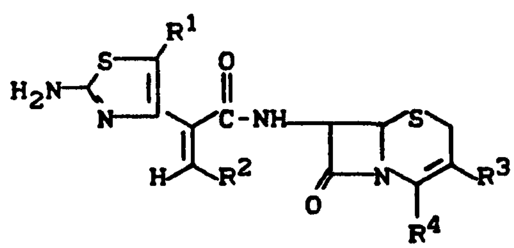

(I),

in welcher

$R^1$ - für Alkyl, Alkenyl, Alkoxy, Alkylthio, Halogen, Trifluormethyl oder Trifluormethoxy steht,

$R^2$ - für Wasserstoff, Alkenyl, für gegebenenfalls substituiertes Alkyl, gegebenenfalls substituiertes Aryl, gegebenenfalls substituiertes Heterocyclyl, Alkoxy, Carboxy, Alkoxycarbonyl, Halogen oder für eine Gruppe der Formel $-S(O)_n-B-A$ steht,

worin

n - 0, 1 oder 2 bedeutet,
B - eine direkte Bindung, Sauerstoff oder eine Gruppe -N-X darstellt, und

A und X unabhängig voneinander für Wasserstoff, gegebenenfalls substituiertes Alkyl, gegebenenfalls substituiertes Aryl oder gegebenenfalls substituiertes Heterocyclyl stehen, oder gemeinsam einen gegebenenfalls substituierten carbocyclischen oder heterocyclischen Ring bilden,

$R^3$ - für Wasserstoff, Alkyl, Alkenyl, Alkoxy, Alkylthio, Halogen oder für eine Gruppe der Formel $-CH_2-Y$ steht,

wobei

Y - für Hydroxy, Formyloxy, Alkanoyloxy, Aminocarbonyloxy, für gegebenenfalls substituiertes Heterocyclylthio, oder für einen stickstoffhaltigen und über N gebundenen positiven geladenen, bis zu insgesamt 3 Stickstoffatome enthaltenden 6-Ring steht, an den bis zu zwei weitere Ringe ankondensiert sein können und der gegebenenfalls substituiert sein kann, oder für eine Gruppe der Formel

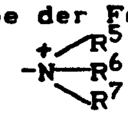

steht, worin

$R^5$,$R^6$ und $R^7$ gleich oder verschieden sein können und einen substituierten oder unsubstituierten Alkylrest oder einen mono- oder bicyclischen, gegebenenfalls substituierten, carbo- oder heterocyclischen Ring bedeuten oder

$R^5$ einen gegebenenfalls substituierten Alkylrest oder einen mono- oder bicyclischen, substituierten oder unsubstituierten, carbo- oder heterocyclischen Ring darstellt und

$R^6$ und $R^7$ gemeinsam mit dem N-Atom einen gegebenenfalls substituierten mono-oder polycyclischen Ring bilden, der gesättigt oder ungesättigt sein kann und Sauerstoff, Schwefel und Stickstoff als weitere Heteroatome enthalten kann

und

$R^4$ - je nach der Bedeutung von $R^3$ für $COO^-$ oder COOH steht,

und deren Salze.

15. Verwendung von Cephalosporinen der allgemeinen Formel (I),

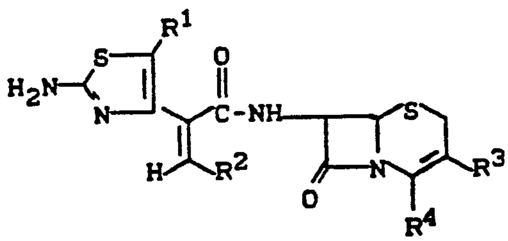

(I),

in welcher

$R^1$ - für Alkyl, Alkenyl, Alkoxy, Alkylthio, Halogen, Trifluormethyl oder Trifluormethoxy steht,

$R^2$ - für Wasserstoff, Alkenyl, für gegebenenfalls substituiertes Alkyl, gegebenenfalls substituiertes Aryl, gegebenenfalls substituiertes Heterocyclyl, Alkoxy, Carboxy, Alkoxycarbonyl, Halogen oder für eine Gruppe der Formel $-S(O)_n-B-A$ steht,

worin

n - 0, 1 oder 2 bedeutet,

B - eine direkte Bindung, Sauerstoff oder eine Gruppe -N-X darstellt, und

A und X unabhängig voneinander für Wasserstoff, gegebenenfalls substituiertes Alkyl, gegebenenfalls substituiertes Aryl oder gegebenenfalls substituiertes Heterocyclyl stehen, oder gemeinsam einen gegebenenfalls substituierten carbocyclischen oder heterocyclischen Ring bilden,

$R^3$ - für Wasserstoff, Alkyl, Alkenyl, Alkoxy, Alkylthio, Halogen oder für eine Gruppe der Formel $-CH_2-Y$ steht,

wobei

Y - für Hydroxy, Formyloxy, Alkanoyloxy, Aminocarbonyloxy, für gegebenenfalls substituiertes Heterocyclylthio, oder für einen stickstoffhaltigen und über N gebundenen positiven geladenen, bis zu insgesamt 3 Stickstoffatome enthaltenden 6-Ring steht, an den bis zu zwei weitere Ringe ankondensiert sein können und der gegebenenfalls substituiert sein kann, oder für eine Gruppe der Formel

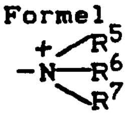

steht, worin

$R^5$, $R^6$ und $R^7$ gleich oder verschieden sein können und einen substituierten oder unsubstituierten Alkylrest oder einen mono- oder bicyclischen, gegebenenfalls substituierten, carbo- oder heterocyclischen Ring bedeuten oder

$R^5$ einen gegebenenfalls substituierten Alkylrest oder einen mono- oder bicyclischen, substituierten oder unsubstituierten, carbo- oder heterocyclischen Ring darstellt und

$R^6$ und $R^7$ gemeinsam mit dem N-Atom einen gegebenenfalls substituierten mono-oder polycyclischen Ring bilden, der gesättigt oder ungesättigt sein kann und Sauerstoff, Schwefel und Stickstoff als weitere Heteroatome enthalten kann

und

$R^4$ - je nach der Bedeutung von $R^3$ für $COO^-$ oder COOH steht,

und deren Salzen bei der Herstellung von Arzneimitteln.